# EUROPEAN PATENT SPECIFICATION

(11) **EP 2 857 411 B1**
(45) Date of publication and mention of the grant of the patent: **06.12.2017**
(21) Application number: 12877493.2
(22) Date of filing: 25.05.2012
(51) Int. Cl.: C07H 15/04, C07H 15/18, C07H 1/00

(54) **METHOD FOR PREPARING FULLY PROTECTION HEPARIN PENTASACCHARIDE AND INTERMEDIATE THEREOF**
VERFAHREN ZUR HERSTELLUNG EINES HEPARIN-PENTASACCHARIDS FÜR VOLLEN SCHUTZ UND ZWISCHENPRODUKT DAVON
PROCÉDÉ DE PRÉPARATION DE PENTASACCHARIDE D'HÉPARINE OFFRANT UNE PROTECTION COMPLÈTE ET SON INTERMÉDIAIRE

(43) Date of publication of application: 08.04.2015
(73) Proprietor: Zhejiang Hisun Pharmaceutical Co. Ltd., Jiaojiang District Taizhou City Zhejiang Province 318000 (CN)
(72) Inventor: DING, Yili, Shanghai 201612 (CN); GUO, Yanghui, Shanghai 201612 (CN); BAI, Hua, Taizhou City Zhejiang 318000 (CN); WU, Yingqiu, Shanghai 201612 (CN); YANG, Xuan, Shanghai 201612 (CN); YAN, Qingyan, Shanghai 201612 (CN); CHAI, Jian, Taizhou City Zhejiang 318000 (CN)
(74) Representative: dompatent von Kreisler Selting Werner - Partnerschaft von Patent- und Rechtsanwälten mbB
(86) International application number: PCT/CN2012/076096
(87) International publication number: WO 2013/174017

(56) References cited:
- WO-A2-2011/014793
- CN-A- 1 558 911
- JP-A- S63 218 691
- US-A1- 2011 003 980
- US-A1- 2012 116 066
- JINQ-CHYI LEE ET AL: "Synthesis of Heparin Oligosaccharides", JOURNAL OF THE AMERICAN CHEMICAL SOCIETY, vol. 126, no. 2, 1 January 2004 (2004-01-01), pages 476-477, XP055212269, ISSN: 0002-7863, DOI: 10.1021/ja038244h
- HU YU-PENG ET AL: "Synthesis of 3-O-sulfonated heparan sulfate octasaccharides that inhibit the herpes simplex virus type 1 host-cell interaction", NATURE CHEMISTRY, NATURE PUBLISHING GROUP, GB, vol. 3, no. 7, 1 July 2011 (2011-07-01), pages 557-563, XP002740202, ISSN: 1755-4330, DOI: 10.1038/NCHEM.1073
- LIN, FENG ET AL.: 'A Facile Preparation of Uronates via Selective Oxidation with TEMPO/KBr/Ca(OCl)Z under Aqueous Conditions' CARBOHYDRATE RESEARCH vol. 339, 2004, pages 1219 - 1223, XP004500607

## Description

### FIELD OF THE INVENTION

The present invention relates to the field of chemical synthesis, and particularly to the preparation of fully protected heparin pentasaccharide and intermediate thereof.

### BACKGROUND OF THE INVENTION

Heparin was first isolated from animal liver by Jay McLean of Johns Hopkins University in 1916, which was then characterized as an active ingredient for anticoagulation (a: Chem. Ind. 1991, 2, 45-50; b: Bull. Johns Hopkins Hosp. 1928, 42, 199). Heparin has the most complex structure in the glycosaminoglycan (GAG) family. Heparin has been clinically used for antithrombosis and cardiovascular diseases for almost 60 years. Its anticoagulation effect is the most deeply investigated and illuminated one among many physiological activities of heparin, which contributes to the general use of low molecular weight heparin (LMWH) as an anti-thrombotic agent, and the replacement of the clinically employed anti-thrombotic agents since 1990s (Blood, 1992,79, 1-17). The process of blood coagulation is the result of a series of sequentially activated coalition factors in plasma, which finally converts the inactive thrombin to be active, so that the soluble fibrinogen can be partially proteolyzed to release insoluble fibrin to result in blood coagulation. Antithrombin III (ATIII) is the inhibitor for a serine proteinase in the process of blood coagulation, especially thrombin IIa and Xa. The reaction between antithrombin III and thrombin has low reaction rate, which, however, increases for thousands of times in the presence of heparin, so that the blood coagulation can be effectively inhibited.

Natural heparin is primarily extracted from animal liver, which is a complex mixture consisting of polysaccharides with different activities. Therefor it is difficult to control the effective dose of natural heparin during use, and thus it has the risk to induce side effects, such as hemorrhage, thrombocytopenia etc. Meanwhile, non-specific binding may occur between heparin and plasma proteins, leading to more complex complications. At the end of 1980s, the effect for antithrombosis treatment was improved due to the discovery of low molecular weight heparin (LMWH). Low molecular weight heparin is obtained by degradation of whole heparin through chemical method, enzymatic method and gamma ray irradiation. However, it is disturbing that both heparin and low molecular weight heparin are animal-originated, so that there will be a potential risk for cross-viral infection among species, which makes its application unsafe. The most effective measure to avoid cross-infection is the application of heparin compounds prepared by chemical synthesis.

The total synthesis of anticoagulant pentasaccharide (Formula I) has been reported by Sinay et al. (Carbohydr. Res. 1984, 132, C5-C9, Carbohydr. Res. 1986, 147. 221 -236) and Boeckel et al. (J. Carbohydr. Chem. 1985, 4, 293-321.) in the middle of 1980s. However, during the process of final deprotection, intermolecular reaction can occur between hemiacetal groups at the reducing end of the pentasaccharide to form stable dimers or trimers, resulting in extremely low reaction efficiency.

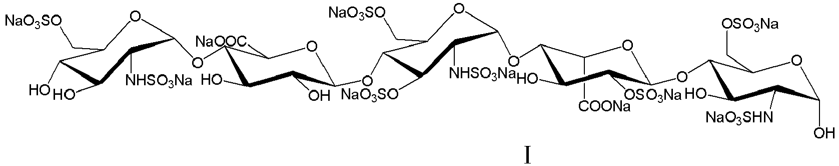

It was found that the formation of dimers or trimers can be avoided in the reducing reaction using methyl end-capped pentasaccharide (Formula II) (Carbohydr. Res. 1987. 167. 67-75), resulting in increased synthesis efficiency. Moreover, it was also suggested by the biological activity test that the biological activity of the heparin pentasaccharide which was protected by methyl at the reducing end was retained.

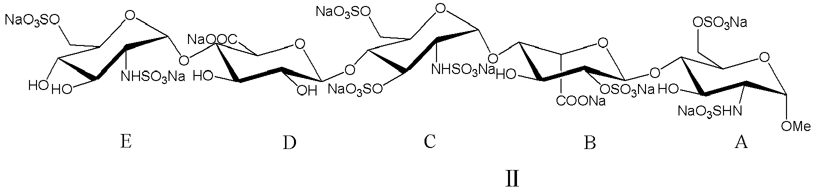

The compound of Formula II has been marketed as an anticoagulant under the common name of Fondaparinux sodium in 2001, and its chemical name is methyl O-(2-Deoxy-6-O-sulfo-2-sulfoamino-α-D-glucopyranosyl)-(1→4)-O-(β-D-glucopyranuronos yl)-(1→4)-O-(2-deoxy-3,6-di-O-sulfo-2-sulfoamino-α-D-glucopyranosyl)-(1→4)-O-(2-O-sul fo-α-L-idopyranuronosyl)-(1→4)-2-deoxy-6-O-sulfo-2-sulfoamino-α-D-glucopyranoside decasodium salt.

The structure of the compound of Formula II has the following characteristics: it is formed by five different monosaccharides sequentially linked by α- or β-glucosidic linkages. Five monosaccharide fragments for the compound of Formula II are expressed by letter A, B, C, D and E sequentially from right to left. In the structure of the pentasaccharide, there are free hydroxy, sulfated hydroxy, and sulfated amino, in which fragment A, C and E are the derivatives of glycosamine, fragment B is the derivative of iduronic acid, and fragment D is the derivative of glucuronic acid. As a result, appropriate protective groups must be considered for the synthesis design, in order to satisfy the following requirements: (1) during the reaction of the formation of glucosidic linkage, the protective group should be favorable for correct formation of the glucosidic linkage in both aspects of regioselectivity and stereoselectivity; (2) the protective group is selected, so that sulfation occurs at required positions, while other hydroxys are not sulfated; (3) due to the extremely long synthetic route of the compound, the selection of the protective group should be beneficial for the reaction efficiency, especially the selectivity and yield of glycosylation.

In the synthesis strategies reported previously, similar protective groups, deprotective groups and modification scheme are employed to achieve the synthesis of Fondaparinux sodium of Formula II. Fondaparinux sodium is synthesized using fully protected pentasaccharide of Formula III, wherein the protective group R₁ is the hydroxy-protecting group which is removed in the first step, and it can be acetyl or benzoyl etc., and the method for the removal of the protective group R₁ should be distinguished from that of the protective group R₂; the protective group R₂ is the hydroxy-protecting group which is removed in the third step, and it can be benzyl; the protective group R₃ is the precursor that should be converted into amino in the third step, and it can be azido or carbobenzoxyamino (-NHCbz).

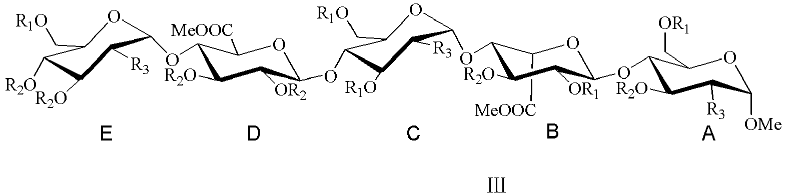

This strategy can be divided into several steps as follows: in the first step, five hydroxy-protecting groups (R1) that are demanded for sulfation in the fully protected pentasaccharide are selectively removed so as to expose them; in the second step, the exposed hydroxy groups are sulfated; in the third step, the remaining 6 hydroxy-protecting groups (R2) are removed so as to expose them, and the amino-protecting groups are removed, or the amino precursors (R3) are converted into amino groups to expose 3 amino groups; in the fourth step, said 3 exposed amino groups are selectively sulfated. The procedure can be shown by conversion of the following structural formulas:

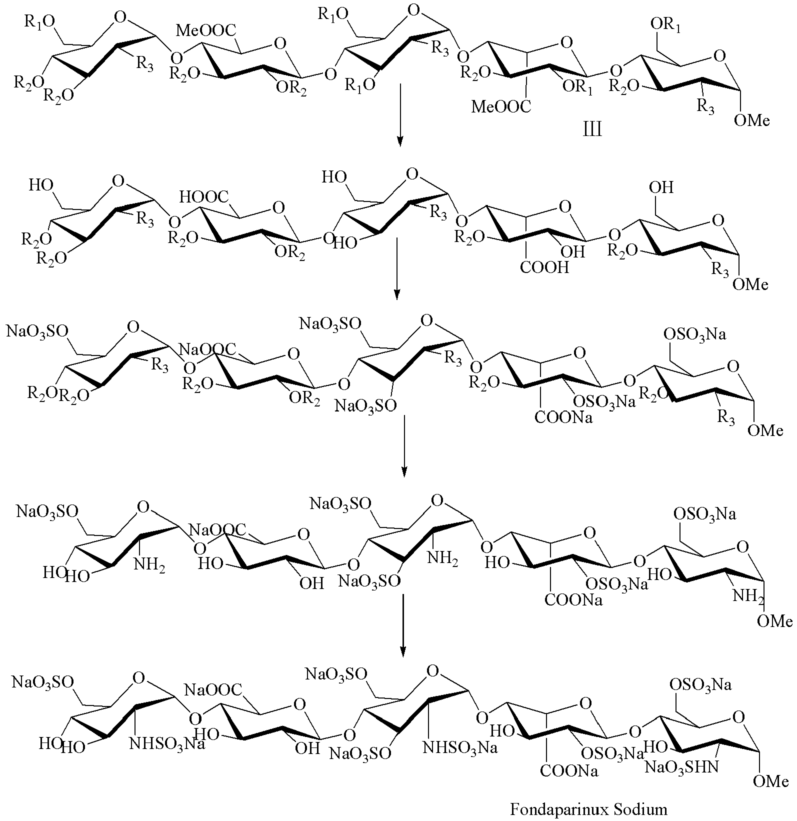

There are some deficiencies in the strategy. Fragment D and fragment C in the structure of Fondaparinux sodium are linked via β-glucosidic linkage. However, during the synthesis of the fully protected pentasaccharide, α-glycosylation and β-glycosylation may both occur in the reaction due to stereoselectivity when fragment D and fragment C are linked, so that α-glucosidic linkage and β-glucosidic linkage are each formed on the anomeric carbon atoms of fragment D. Attempts are made to solve this problem.

As reported by Sinay et al. (Carbohydrate Research, 1984, 132, C5-C9) in 1984, a DC disaccharide coupled via β-glucosidic linkage is synthesized in 6 days with a yield of 50%. The same result is obtained in United States Patent US 4818816. In 1991, Sanofi reported a similar result, in which the yield was 51%, and the α/β ratio on the anomeric carbons of fragment D is 1/12 (Bioorg. Med. Chem. Lett., 1991, 1, 2, 99-102). This procedure has disadvantages such as low yield, difficulty in purification, and the presence of anomers will also make the purification step difficult. Subsequently, another method was proposed by Kuzuhara (Carbohydrate Research, 1985, 141, 273) and simplified by Petitou, in which disaccharide fragment DC was synthesized by some chemical conversion steps using cellobiose as the starting material for fragment DC directly (Bioorg. Med. Chem. Lett., 1991, 1, 2, 95-98). In this method, the existing β-(1→4) glucosidic linkage in cellobiose is used, so that the step of glycosylation can be avoided. However, this method also has drawbacks, such as complex operation steps for the protective group, long synthesis route, low overall yield and difficulty in purification.

A solution has been proposed in United States Patent US 7541445 of Alchemia company, in which benzoyl is used as the 2-hydroxy-protecting group of fragment D, so that when coupled to fragment C, it is capable of controlling the stereoselectivity of the coupling reaction to be β-configuration as "neighboring group participation" protective group, and the benzoyl protective group can be removed and replaced by benzyl at the stage of trisaccharide fragment EDC. However, in this method, acyl protective group can not be used on fragment E and fragment C, and only *p*-methoxybenzyl protective groups can be used, so that after the formation of fully protected pentasaccharide by all coupling reactions, the p-methoxybenzyl protective groups are removed. Moreover, there are two obvious disadvantages for this method, in which the first one is that the glycosylation reaction can be influenced by the *p*-methoxybenzyl protecting groups on fragment E and fragment C, and the influence become higher in later period, especially at the time of glycosylation reaction of trisaccharide fragment EDC and disaccharide fragment BA, with extremely low yield and significant difficulty in purification; the second one is that after the synthesis of the fully protected pentasaccharide, one more step for the removal of p-methoxybenzyl is required, and at later stage of synthesis, this added step will have huge impact on the reaction yield of the route and the convenience of operation. As a result, it is important to establish a synthesis process with high yield and simple purification methods.

### SUMMARY OF THE INVENTION

One of the objectives of the present invention is to provide a novel intermediate for preparing fully protected heparin pentasaccharide and the preparation method thereof. The second objective of the present invention is to provide a method for preparing fully protected heparin pentasaccharide.

The present invention provides a novel intermediate for preparing fully protected heparin pentasaccharide, which has the following structure of Formula V: 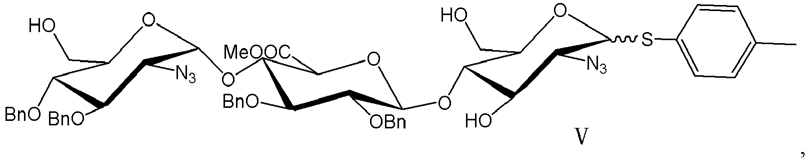 wherein, the configuration of the monosaccharide unit and the stereochemistry of the linkages among each monosaccharides are expressed as D-glucose-α-1,4-D-glucuronic acid-β-1,4-D-glucose.

The present invention also provides another novel intermediate for preparing fully protected heparin pentasaccharide, which has the following structure of Formula VI: 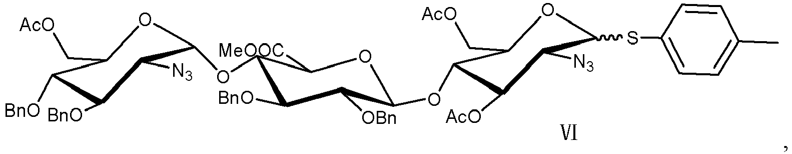 wherein, the configuration of the monosaccharide unit and the stereochemistry of the linkages among each monosaccharides are expressed as D-glucose-α-1,4-D-glucuronic acid-β-1,4-D-glucose.

The present invention provides a method for preparing the compound of formula V, in which the compound of formula V is obtained by removing 3 *p*-methoxybenzyl groups from the compound of formula IV, and the reaction process is shown as follows: 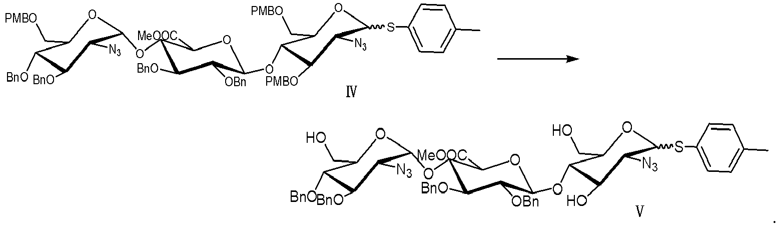 wherein, in specific embodiments, the reagent used in the reaction for removing three *p*-methoxybenzyl groups can be 2,3-dichloro-5,6-dicyano-1,4-benzoquinone, ammonium ceric nitrate or strong protonic acid. Furthermore, the strong protonic acid is, for example, hydrochloric acid, trifluoroacetic acid etc.

In the above reaction, the compound of Formula IV can be commercially available, or can be prepared according to the method in United States Patent US 7541445.

The present invention also provides a method for preparing the compound of formula VI, in which the compound of Formula VI is obtained by reacting the compound of formula V with an acetylation reagent under alkaline condition in order to protect the three exposed hydroxy groups with the acetyl group, and the reaction process is shown as follows:

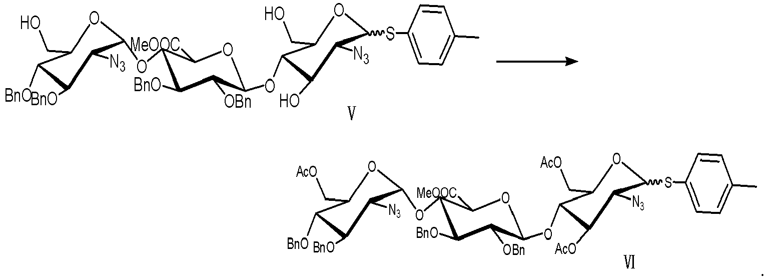

The base used in the reaction can be an organic base or an inorganic base. The organic base is preferably selected from pyridine, triethylamine etc., and the inorganic base is preferably selected from potassium carbonate, sodium carbonate, sodium bicarbonate etc. The acetylation reagent used in the reaction is preferably selected from acetic anhydride, acetyl chloride etc.

The present invention provides a method for preparing the compound of Formula XVIII, in which the compound of Formula XVIII, a fully protected pentasaccharide, is obtained by the glycosylation reaction of the compound of Formula VI and the compound of Formula X under the condition that allows the formation of glucosidic linkage, and the reaction process is shown as follows:

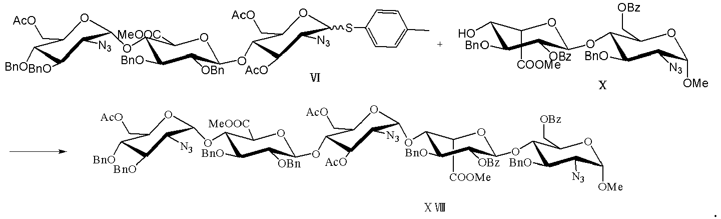

The reagent for the formation of glucosidic linkage is preferably selected from N-iodosuccinimide-trifluoromethanesulfonic acid, N-iodosuccinimide-silver trifluoromethanesulfonate, trifluoromethanesulfonate etc.

Fondaparinux sodium can be further synthesized using the compound of Formula XVIII by reference to the method reported (Carbohydrate Research, 1987, 167, 67).

In further embodiments, the compound of Formula X can be prepared through the following method, in which the compound of Formula X is obtained by the methyl esterification of the compound of Formula IX using a methylation reagent, and the reaction process is shown as follows:

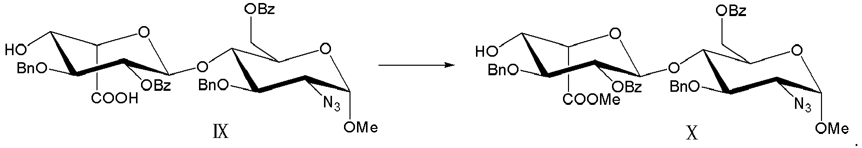

The methylation reagent used in the reaction is preferably selected from diazomethane, (trimethylsilyl)diazomethane, iodomethane, bromomethane, chloromethane etc.

In further embodiments, the compound of Formula IX can be prepared through the following method, in which the compound of Formula IX is obtained by converting the exposed primary alcoholic hydroxyl group in the compound of Formula VIII into carboxyl via oxidation in the presence of an oxidizing agent and a catalyst, and the reaction process is shown as follows:

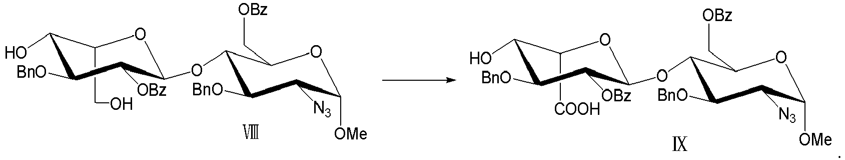

The oxidizing agent used in the reaction is preferably selected from iodobenzene diacetate, calcium hypochlorite, sodium hypochlorite etc., and the catalyst used in the reaction is preferably 2,2,6,6-tetramethyl-1-piperidinyloxy.

In further embodiments, the compound of Formula VIII can be prepared through the following method, in which the compound of Formula VIII is obtained by removing the acetyl-protecting group of the compound of Formula VII in the presence of a deacetylation reagent, and the reaction process is shown as follows:

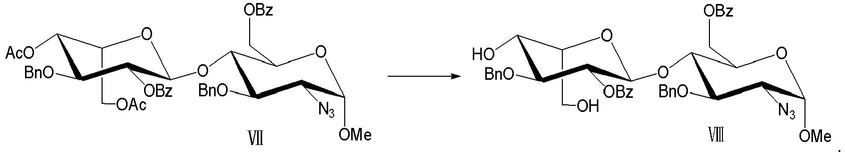

The deacetylation reagent used in the reaction is preferably selected from a solution of hydrogen chloride in methanol, a solution of hydrogen chloride in ethanol, a solution of triethylamine in methanol, a solution of triethylamine in ethanol, sodium methoxide or sodium ethoxide.

In further embodiments, the compound of Formula VII can be prepared through the following method, in which the compound of Formula VII, a disaccharide, is obtained by the glycosylation reaction of the compound of Formula XI and the compound of Formula XII under the condition that allows the formation of glucosidic linkage, and the reaction process is shown as follows:

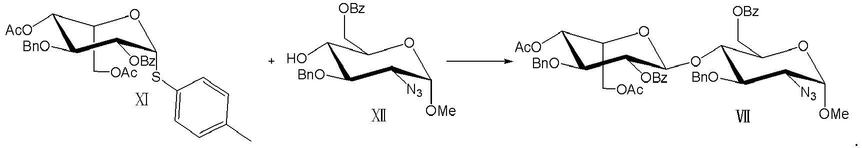

The reagent for the formation of glucosidic linkage is preferably selected from N-iodosuccinimide-trifluoromethanesulfonic acid, N-iodosuccinimide-silver trifluoromethanesulfonate, trifluoromethanesulfonate etc.

In further embodiments, the compound of Formula XI can be prepared through the following method, in which the compound of Formula XI is obtained by reacting the compound of formula XIII with an acetylation reagent under alkaline condition in order to protect the 2 exposed hydroxy groups with the acetyl group, and the reaction process is shown as follows:

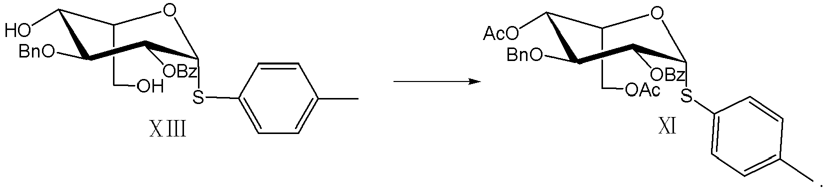

The base used in the reaction can be an organic base or an inorganic base. The organic base is preferably selected from pyridine, triethylamine etc., and the inorganic base is preferably selected from potassium carbonate, sodium carbonate, sodium bicarbonate etc. The acetylation reagent used in the reaction is preferably selected from acetic anhydride, acetyl chloride etc.

In the above reaction, the compound of Formula XIII can be commercially available, or can be prepared according to the method in United States Patent US 7541445.

In further embodiments, the compound of Formula XII can be prepared by the following method, which comprises the following steps:
1) the compound of Formula XIV is treated by a strong base in order to give the compound of Formula XV by removing the carbobenzoxy group (-Cbz), and exposing the amino group, and the reaction process is shown as follows: 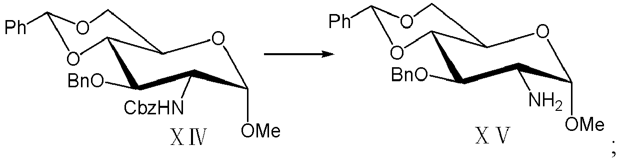
   The base used in the reaction is preferably selected from sodium hydroxide, potassium hydroxide, calcium hydroxide or barium hydroxide.
   The compound of Formula XIV can be commercially available, or can be prepared according to the method in Carbohydrate Research, 1984, 130, 221.
2) the compound of Formula XV is reacted with a diazo transfer reagent in order to give the compound of Formula XVI by converting the exposed amino groups in the compound of Formula XV into the azido group, and the reaction process is shown as follows: 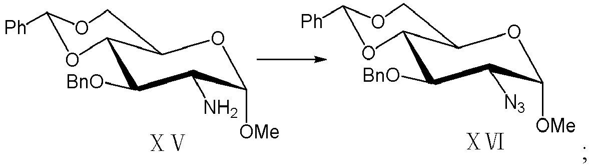
   The diazo transfer reagent used in the reaction is preferably selected from trifluoromethanesulfonyl azide or 1H-imidazole-1-sulfonyl azide hydrochloride;
3) the benzylidene is removed from the compound of Formula XVI under acidic condition to give the compound of Formula XVII, and the reaction process is shown as follows: 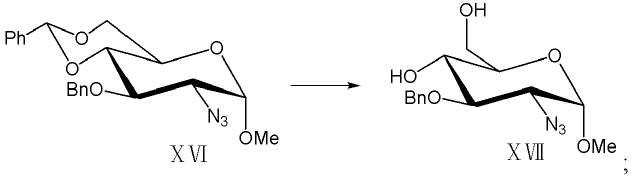
   The acid used in the reaction is preferably selected from acetic acid, trifluoroacetic acid or *p*-toluenesulfonic acid;
4) the compound of Formula XVII is reacted with a benzoylation reagent under alkaline condition to give the compound of Formula XII by protecting the primary alcoholic hydroxyl group with the benzoyl group, and the reaction process is shown as follows:

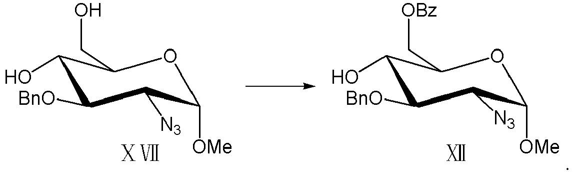

The base used in the reaction can be an organic base or an inorganic base. The organic base is preferably selected from pyridine, triethylamine etc., and the inorganic base is preferably selected from potassium carbonate, sodium carbonate, sodium bicarbonate etc. The benzoylation reagent used in the reaction is preferably selected from benzoic anhydride, benzoyl chloride etc.

The present invention also provides another novel intermediate for preparing fully protected heparin pentasaccharide, which has the following structure of Formula IX: 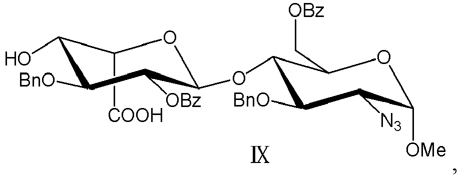 wherein, the configuration of the monosaccharide unit and the stereochemistry of the linkages among each monosaccharides are expressed as L-idoronic acid-α-1,4-D-glucose.

The present invention provides a method for preparing the compound of Formula IX, in which the compound of Formula IX is obtained by converting the exposed primary alcoholic hydroxyl group in the compound of Formula VIII into carboxyl via oxidation in the presence of an oxidizing agent and a catalyst, and the reaction process is shown as follows:

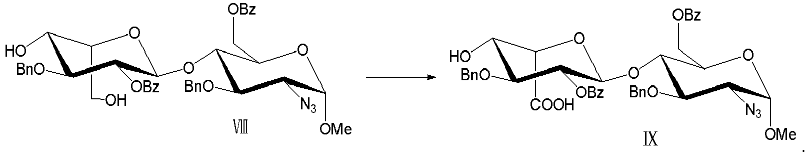

The oxidizing agent used in the reaction is preferably selected from iodobenzene diacetate, calcium hypochlorite, sodium hypochlorite etc., and the catalyst used in the reaction is preferably 2,2,6,6-tetramethyl-1-piperidinyloxy etc.

The present invention also provides another novel intermediate for preparing fully protected heparin pentasaccharide, which has the following structure of Formula VII: 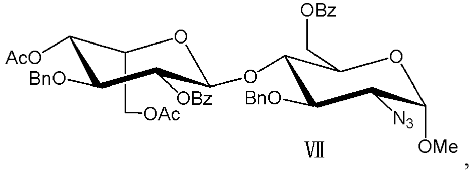 wherein, the configuration of the monosaccharide unit and the stereochemistry of the linkages among each monosaccharides are expressed as L-idose-α-1,4-D-glucose.

The present invention also provides a method for preparing the compound of Formula VII, in which the compound of Formula VII, a disaccharide, is obtained by the glycosylation reaction of the compound of Formula XI and the compound of Formula XII under the condition that allows the formation of glucosidic linkage, and the reaction process is shown as follows:

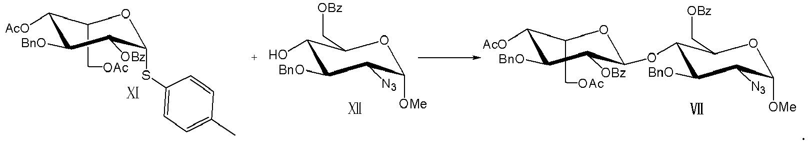

The reagent for the formation of glucosidic linkage is preferably selected from N-iodosuccinimide-trifluoromethanesulfonic acid, N-iodosuccinimide-silver trifluoromethanesulfonate, trifluoromethanesulfonate etc.

The present invention also provides another novel intermediate for preparing fully protected heparin pentasaccharide, which has the following structure of Formula XI:

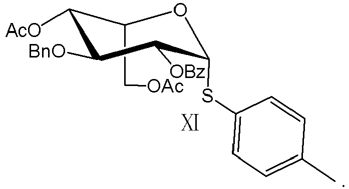

The present invention provides a method for preparing the compound of Formula XI, in which the compound of Formula XI is obtained by reacting the compound of formula XIII with an acetylation reagent under alkaline condition in order to protect the 2 exposed hydroxy groups with the acetyl group, and the reaction process is shown as follows:

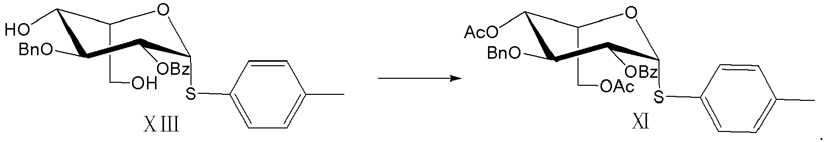

The base used in the reaction can be an organic base or an inorganic base. The organic base is preferably selected from pyridine, triethylamine etc., and the inorganic base is preferably selected from potassium carbonate, sodium carbonate, sodium bicarbonate etc. The acetylation reagent used in the reaction is preferably selected from acetic anhydride, acetyl chloride etc.

The present invention also provides a method for preparing the compound of Formula XII, which comprises the following steps:
1) the compound of Formula XIV is treated by a strong base in order to give the compound of Formula XV by removing the carbobenzoxy group (-Cbz), and exposing the amino group, and the reaction process is shown as follows: 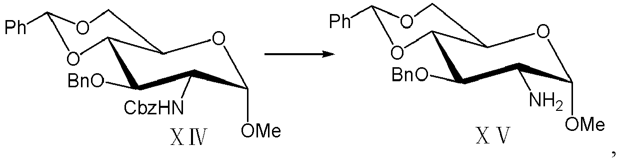
   The base used in the reaction is preferably selected from sodium hydroxide, potassium hydroxide, calcium hydroxide or barium hydroxide. The compound of Formula XIV can be commercially available, or can be prepared according to the method in Carbohydrate Research, 1984, 130, 221.
2) the compound of Formula XV is reacted with a diazo transfer reagent in order to give the compound of Formula XVI by converting the exposed amino groups in the compound of Formula XV into the azido group, and the reaction process is shown as follows: 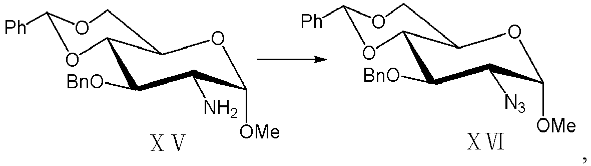
   The diazo transfer reagent used in the reaction is preferably selected from trifluoromethanesulfonyl azide or 1H-imidazole-1-sulfonyl azide hydrochloride;
3) the benzylidene is removed from the compound of Formula XVI under acidic condition to give the compound of Formula XVII, and the reaction process is shown as follows: 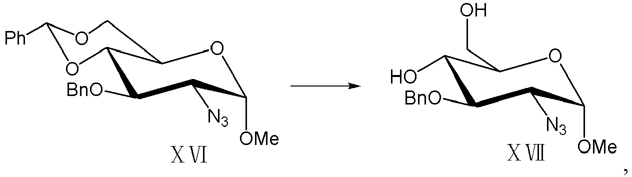
   The acid used in the reaction is preferably selected from acetic acid, trifluoroacetic acid or *p*-toluenesulfonic acid;
4) the compound of Formula XVII is reacted with a benzoylation reagent under alkaline condition to give the compound of Formula XII by protecting the primary alcoholic hydroxyl group with the benzoyl group, and the reaction process is shown as follows:

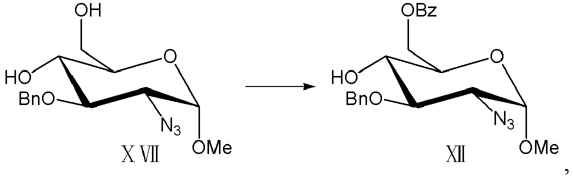

The base used in the reaction can be an organic base or an inorganic base. The organic base is preferably selected from pyridine, triethylamine etc., and the inorganic base is preferably selected from potassium carbonate, sodium carbonate, sodium bicarbonate etc. The benzoylation reagent used in the reaction is preferably selected from benzoic anhydride, benzoyl chloride etc.

As compared to the prior art, the present invention possesses the following advantages:
In the present invention, three p-methoxybenzyl protecting groups in the trisaccharide fragment EDC are converted into the acetyl protecting group, followed by the glycosylation of the trisaccharide fragment EDC and the disaccharide fragment BA, so that the problems due to the instability of the p-methoxybenzyl protecting group under the glycosylation condition, such as low yield and difficulty in purification, are solved, and the fully protected heparin pentasaccharide is prepared by the glycosylation of the trisaccharide fragment EDC and the disaccharide fragment BA with high yield and simple purification.

Moreover, the present invention provides a new method for preparing the compound of Formula XII, i.e., monosaccharide fragment A, so that during the synthesis process of the monosaccharide fragment A, the amount of diazo transfer reagent, which is expensive and toxic, is significantly reduced, and as well, the detrimental effect of the carbobenzoxy amino group (CbzNH-) on the glycosylation is avoided. In the process of synthesis, the intermediate can be purified simply by recrystallization, which is simple, cheap and suitable for the large scale production of the monosaccharide fragment A.

The synthesis process of the monosaccharide fragment B, i.e., the compound of Formula XI, used in the present invention is simple, cheap and avoids the use of expensive and toxic reagent.

The present invention further provides a new method for synthesizing the disaccharide fragment BA of Formula VII, in which the yield of the glycosylation between 2 monosaccharide fragments B and fragment A is high, and the purification is simple. Furthermore, in the process of disaccharide conversion after the glycosylation reaction, the acetyl is selectively removed based on the difference between acetyl and benzoyl; subsequently, the exposed primary alcoholic hydroxyl group is selectively oxidized into carboxyl, followed by methyl esterification to give the disaccharide fragment BA. The synthesis route in the present invention is short, with good selectivity and high yield.

### DETAILED EMBODIMENTS

The methods for preparing the fully protected heparin pentasaccharide and the intermediate thereof are disclosed in the Examples of the present invention. It can be implemented by properly modifying the processing parameters by the one of skill in the art with reference to the content herein. Particularly, it should be noted that all similar replacements and modifications are apparent to the one of skill in the art, all of which are regarded to be included in the present invention. The present invention has been described by preferred Examples, and it is apparent that modification, or proper change and the combination thereof can be made to the products and methods described herein by those skilled in the art, without departing from the content, spirit and scope of the invention, in order to achieve and apply the techniques disclosed in the present invention.

In order to make the present invention better understood, the present invention will be described in detail with reference to the Examples below.

### Example 1: Preparation of p-methylphenyl (2-azido-3,4-di-O-benzyl-2-deoxy-α-D-glucopyranosyl)-(1→4)-O-(methyl 2,3-di-O-benzyl-β-D-glucopyranosyluronate)-(1→4)-O-2-azido-2-deoxy-1-thio-α/β-D-glucop yranoside (V)

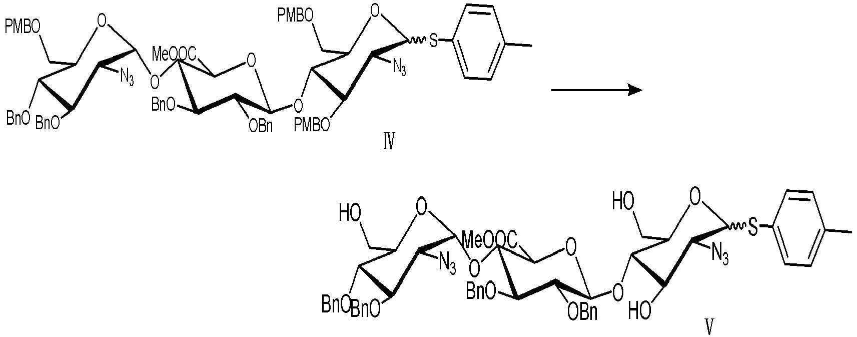

8.4 g compound of Formula IV was dissolved in a mixture of 80 ml dichloromethane and 8 ml water, and cooled in an ice water bath, into which 6.0 g 2,3-dichloro-5,6-dicyano-1,4-benzoquinone was added. After the reaction was finished under spontaneous temperature increase, it was quenched by adding saturated NaHCO₃ solution. The mixture stood for phase separation, in which the water phase was extracted once by dichloromethane. The organic phases were combined, washed sequentially by saturated NaHCO₃ solution and saturated saline solution, dried and concentrated. 5.0 g product was obtained after purification by silica gel column chromatography. In order to confirm the structure of the compound, the compound of Formula V of α configuration was obtained by further purification using silica gel column chromatography, and its structure was characterized.

The MS, and ¹H NMR data for the compound of Formula V of α configuration: ESI-MS(m/z): 1066[M+NH₄]⁺, 1021[M-N₂+H]⁺; ¹H NMR(400 MHz, CDCl₃): δ 7.37-7.25(m, 22 H), 7.13(d, J = 8.0 Hz, 2 H), 5.51(d, J = 3.6 Hz, 1 H), 5.42(d, J = 5.6 Hz, 1 H), 4.96(d, J = 10.8 Hz, 1 H), 4.89-4.76(m, 6 H), 4.65(d, J = 10.8 Hz, 1 H), 4.58(d, J = 8.0 Hz, 1 H), 4.18-4.06(m, 3 H), 3.94-3.63(m, 10 H), 3.79(s, 3 H), 3.54(t, J = 8.4 Hz, 2 H), 3.44(dt, J₁ = 10.0 Hz, J₂ = 2.8 Hz, 1 H), 3.25(dd, J₁ = 10.4 Hz, J₂ = 4.0 Hz, 1 H), 2.33(s, 3 H), 1.67(dd, J₁ = 8.8 Hz, J₂ = 4.8 Hz, 1 H), 1.58(dd, J₁ = 8.8 Hz, J₂ = 4.4 Hz, 1 H).

### Example 2: Preparation of p-methylphenyl (2-azido-3,4-di-O-benzyl-2-deoxy-α-D-glucopyranosyl)-(1→4)-O-(methyl 2,3-di-O-benzyl-β-D-glucopyranosyluronate)-(1→4)-O-2-azido-2-deoxy-1-thio-α/β-D-glucop yranoside (V)

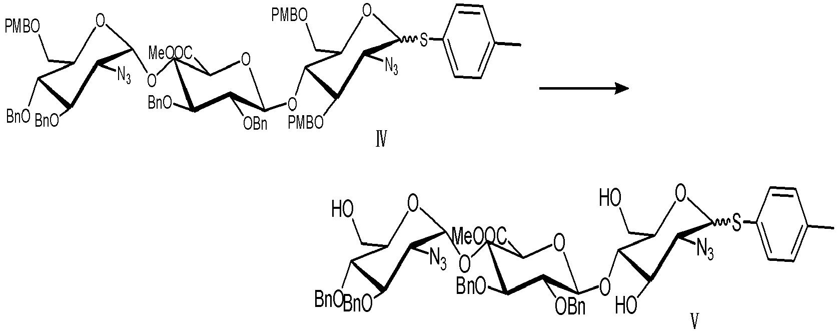

3.8 g compound of Formula IV was dissolved in a mixture of 40 ml acetonitrile and 2 ml water, and cooled in an ice water bath, into which 6.6 g ammonium ceric nitrate was added. The reaction temperature was maintained until it was quenched by adding saturated NaHCO₃ solution. It was extracted by dichloromethane twice, and the organic phases were combined, washed sequentially by saturated NaHCO₃ solution and saturated saline solution, dried and concentrated. 2.1 g product was obtained after purification by silica gel column chromatography. In order to confirm the structure of the compound, the compound of Formula V of α configuration was obtained by further purification using silica gel column chromatography, and its structure was characterized.

The MS, and ¹H NMR data for the compound of Formula V of α configuration: ESI-MS(m/z): 1066[M+NH₄]⁺, 1021[M-N₂+H]⁺; ¹H NMR(400 MHz, CDCl₃): δ 7.37-7.25(m, 22 H), 7.13(d, J = 8.0 Hz, 2 H), 5.51(d, J = 3.6 Hz, 1 H), 5.42(d, J = 5.6 Hz, 1 H), 4.96(d, J = 10.8 Hz, 1 H), 4.89-4.76(m, 6 H), 4.65(d, J = 10.8 Hz, 1 H), 4.58(d, J = 8.0 Hz, 1 H), 4.18-4.06(m, 3 H), 3.94-3.63(m, 10 H), 3.79(s, 3 H), 3.54(t, J = 8.4 Hz, 2 H), 3.44(dt, J₁ = 10.0 Hz, J₂ = 2.8 Hz, 1 H), 3.25(dd, J₁ = 10.4 Hz, J₂ = 4.0 Hz, 1 H), 2.33(s, 3 H), 1.67(dd, J₁ = 8.8 Hz, J₂ = 4.8 Hz, 1 H), 1.58(dd, J₁ = 8.8 Hz, J₂ = 4.4 Hz, 1 H).

### Example 3: Preparation of p-methylphenyl (2-azido-3,4-di-O-benzyl-2-deoxy-α-D-glucopyranosyl)-(1→4)-O-(methyl 2,3-di-O-benzyl-β-D-glucopyranosyluronate)-(1→4)-O-2-azido-2-deoxy-1-thio-α/β-D-glucop yranoside (V)

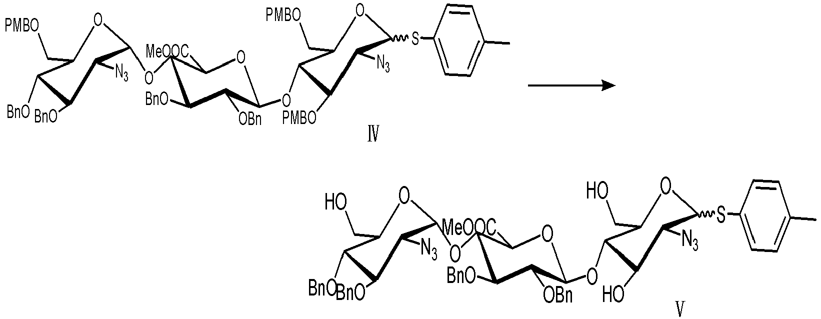

0.92 g compound of Formula IV was dissolved in 10 ml dichloromethane, and cooled in an ice water bath, into which 1 ml trifluoroacetic acid was added. The reaction temperature was maintained until it was finished by neutralizing the reaction mixture with saturated NaHCO₃ solution. It was extracted by dichloromethane twice, and the organic phases were combined, washed sequentially by saturated NaHCO₃ solution and saturated saline solution, dried and concentrated. 0.48 g product was obtained after purification by silica gel column chromatography. In order to confirm the structure of the compound, the compound of Formula V of α configuration was obtained by further purification using silica gel column chromatography, and its structure was characterized.

The MS, and ¹H NMR data for the compound of Formula V of α configuration: ESI-MS(m/z): 1066[M+NH₄]⁺, 1021[M-N₂+H]⁺; ¹H NMR(400 MHz, CDCl₃): δ 7.37-7.25(m, 22 H), 7.13(d, J = 8.0 Hz, 2 H), 5.51(d, J = 3.6 Hz, 1 H), 5.42(d, J = 5.6 Hz, 1 H), 4.96(d, J = 10.8 Hz, 1 H), 4.89-4.76(m, 6 H), 4.65(d, J = 10.8 Hz, 1 H), 4.58(d, J = 8.0 Hz, 1 H), 4.18-4.06(m, 3 H), 3.94-3.63(m, 10 H), 3.79(s, 3 H), 3.54(t, J = 8.4 Hz, 2 H), 3.44(dt, J₁ = 10.0 Hz, J₂ = 2.8 Hz, 1 H), 3.25(dd, J₁ = 10.4 Hz, J₂ = 4.0 Hz, 1 H), 2.33(s, 3 H), 1.67(dd, J₁ = 8.8 Hz, J₂ = 4.8 Hz, 1 H), 1.58(dd, J₁ = 8.8 Hz, J₂ = 4.4 Hz, 1 H).

### Example 4: Preparation of p-methylphenyl (6-O-acetyl-2-azido-3,4-di-O-benzyl-2-deoxy-α-D-glucopyranosyl)-(1→4)-O-(methyl 2,3-di-O-benzyl-β-D-glucopyranosyluronate)-(1→4)-O-3,6-di-O-acetyl-2-azido-2-deoxy-1-th io-α/β-D-glucopyranoside (VI)

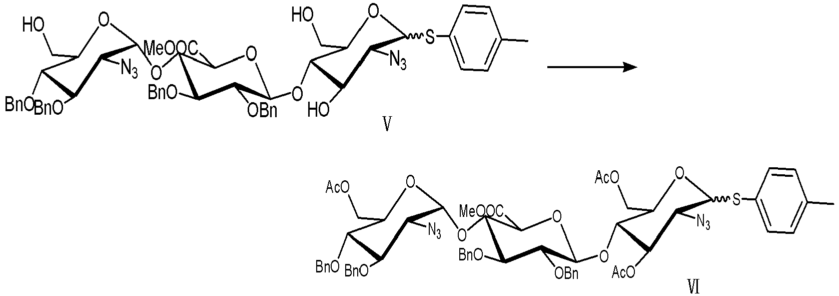

7.9 g compound of Formula V was dissolved in 100 ml dichloromethane, into which 6.3 ml pyridine and 0.3 g *p*-dimethyl aminopyridine were added. After cooled in an ice water bath, 7.7 ml acetic anhydride was added dropwise. When the addition was finished, the reaction was spontaneously warmed up to room temperature until the reaction was completed. The pH was adjusted to weak alkalinity by saturated sodium bicarbonate solution. The organic phase was removed, and the water phase was extracted by dichloromethane. The organic phases were combined, and washed sequentially by 10% HCl solution, water and saturated saline solution. The organic phase was separated, dried over anhydrous sodium sulfate, and concentrated. 7.9 g product was obtained by silica gel column chromatography. In order to confirm the structure of the compound, the compound of Formula VI of α configuration was obtained by further purification using silica gel column chromatography, and its structure was characterized.

The MS, and ¹H NMR data for the compound of Formula VI of α configuration: ESI-MS(m/z): 1192[M+NH₄]⁺, 1147[M-N₂+H]⁺; ¹H NMR(400 MHz, CDCl₃): δ 7.37-7.23(m, 22 H), 7.11(d, J = 7.6 Hz, 2 H), 5.51(d, J = 40 Hz, 1 H), 5.46(d, J = 5.6 Hz, 1 H), 5.31(t, J = 10.0 Hz, 1 H), 4.99(d, J = 10.8 Hz, 1 H), 4.87-4.80(m, 5 H), 4.75(d, J = 11.6 Hz, 1 H), 4.55(d, J = 11.6 Hz, 1 H), 4.38-4.35(m, 1 H), 4.32(d, J = 8.0 Hz, 1 H), 4.28-4.17(m, 4 H), 4.06(t, J = 9.2 Hz, 1 H), 3.94(dd, J₁ = 10.8 Hz, J₂ = 5.6 Hz, 1 H), 3.87-3.84(m, 2 H), 3.76(s, 3 H), 3.74-3.65(m, 2 H), 3.51-3.44(m, 3 H), 3.27(dd, J₁ = 10.4 Hz, J₂ = 4.0 Hz, 1 H), 2.33(s, 3 H), 2.08(s, 3 H), 2.03(s, 3 H), 2.00(s, 3 H).

### Example 5: Preparation of p-methylphenyl (6-O-acetyl-2-azido-3,4-di-O-benzyl-2-deoxy-α-D-glucopyranosyl)-(1→4)-O-(methyl 2,3-di-O-benzyl-β-D-glucopyranosyluronate)-(1→4)-O-3,6-di-O-acetyl-2-azido-2-deoxy-1-th io-α/β-D-glucopyranoside (VI)

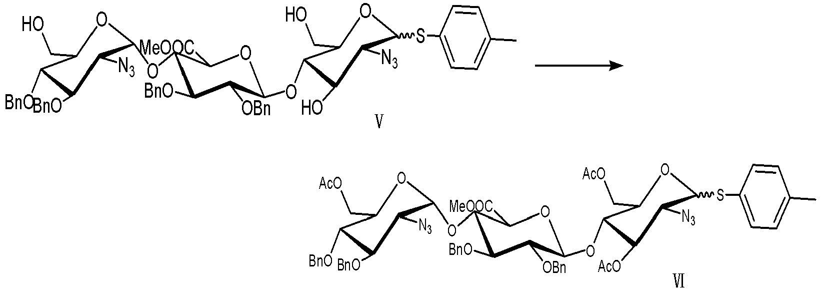

3.38 g compound of Formula V was dissolved in 20 ml acetonitrile, into which 2.05 g sodium carbonate and 1.64 g acetic anhydride were added. The mixture was stirred at room temperature until the reaction was finished. Excessive sodium carbonate was neutralized by dilute hydrochloric acid, and it was filtered and concentrated. The oil mixture was dissolved in ethyl acetate, and washed by saturated saline solution. Subsequently, it was dried over anhydrous sodium sulfate, and concentrated. 3.1 g product was obtained by silica gel column chromatography. In order to confirm the structure of the compound, the compound of Formula VI of α configuration was obtained by further purification using silica gel column chromatography, and its structure was characterized.

The MS, and ¹H NMR data for the compound of Formula VI of α configuration: ESI-MS(m/z): 1192[M+NH₄]⁺, 1147[M-N₂+H]⁺; ¹H NMR(400 MHz, CDCl₃): δ 7.37-7.23(m, 22 H), 7.11(d, J = 7.6 Hz, 2 H), 5.51(d, J = 40 Hz, 1 H), 5.46(d, J = 5.6 Hz, 1 H), 5.31(t, J = 10.0 Hz, 1 H), 4.99(d, J = 10.8 Hz, 1 H), 4.87-4.80(m, 5 H), 4.75(d, J = 11.6 Hz, 1 H), 4.55(d, J = 11.6 Hz, 1 H), 4.38-4.35(m, 1 H), 4.32(d, J = 8.0 Hz, 1 H), 4.28-4.17(m, 4 H), 4.06(t, J = 9.2 Hz, 1 H), 3.94(dd, J₁ = 10.8 Hz, J₂ = 5.6 Hz, 1 H), 3.87-3.84(m, 2 H), 3.76(s, 3 H), 3.74-3.65(m, 2 H), 3.51-3.44(m, 3 H), 3.27(dd, J₁ = 10.4 Hz, J₂ = 4.0 Hz, 1 H), 2.33(s, 3 H), 2.08(s, 3 H), 2.03(s, 3 H), 2.00(s, 3 H).

### Example 6: Preparation of p-methylphenyl 4,6-di-O-acetyl-2-O-benzoyl-3-O-benzyl-1-thio-β-L-idosopyranoside (XI)

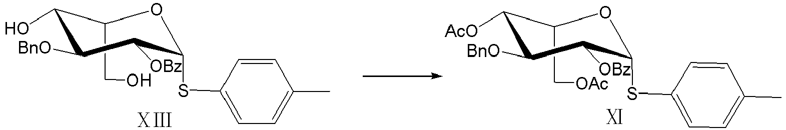

476 g compound of Formula XIII was dissolved in 4.5 L dichloromethane, into which 470 ml pyridine and 12 g *p*-dimethyl aminopyridine were sequentially added. After cooled in an ice water bath, 234 ml acetic anhydride was slowly added dropwise. When the addition was finished, the reaction was spontaneously warmed up to room temperature until the reaction was completed. Subsequently, low-boiling point solvent was removed by concentration under reduced pressure. The concentrated solution was diluted by dichloromethane, and washed sequentially by water, 10% citric acid solution, saturated sodium bicarbonate solution, and saturated sodium chloride solution. It was then dried, concentrated, and purified by column chromatography to give 440 g white solid.

The MS, and ¹H NMR data for the compound of Formula XI: ESI-MS(m/z): 587[M+Na]⁺; ¹H NMR(400 MHz, CDCl₃): δ 8.11(d, J = 7.6 Hz, 2 H), 7.62-7.34(m, 10 H), 7.16(d, J = 8.0 Hz, 2 H), 5.61(s, 1 H), 5.50(s, 1 H), 5.18-5.15(m, 1 H), 5.04(s, 1 H), 4.97(d, J = 12.0 Hz, 1 H), 4.81(d, J = 12.0 Hz, 1 H), 4.38-4.28(m, 2 H), 3.96(s, 1 H), 2.36(s, 3 H), 2.11(s, 3 H), 1.99(s, 3 H).

### Example 7: Preparation of p-methylphenyl 4,6-di-O-acetyl-2-O-benzoyl-3-O-benzyl-1-thio-β-L-idosopyranoside (XI)

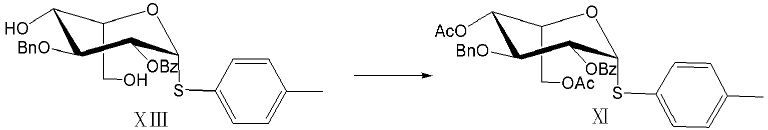

15.5 g compound of Formula XIII was dissolved in 200 ml acetonitrile, into which 30.75 g sodium carbonate and 24.6 g acetic anhydride were sequentially added. The mixture was stirred at room temperature until the reaction was finished.. Excessive sodium carbonate was neutralized by dilute hydrochloric acid, and it was filtered and concentrated. The oil mixture was dissolved in ethyl acetate, and washed by saturated saline solution. Subsequently, it was dried over anhydrous sodium sulfate, and concentrated.. 11.6 g white solid was obtained by purification using column chromatography.

The MS, and ¹H NMR data for the compound of Formula XI: ESI-MS(m/z): 587[M+Na]⁺; ¹H NMR(400 MHz, CDCl₃): δ 8.11(d, J = 7.6 Hz, 2 H), 7.62-7.34(m, 10 H), 7.16(d, J = 8.0 Hz, 2 H), 5.61(s, 1 H), 5.50(s, 1 H), 5.18-5.15(m, 1 H), 5.04(s, 1 H), 4.97(d, J = 12.0 Hz, 1 H), 4.81(d, J = 12.0 Hz, 1 H), 4.38-4.28(m, 2 H), 3.96(s, 1 H), 2.36(s, 3 H), 2.11(s, 3 H), 1.99(s, 3 H).

### Example 8: methyl 2-amino-3-O-benzyl-4,6-O-benzylidene-2-deoxy-α-D-glucopyranoside (XV)

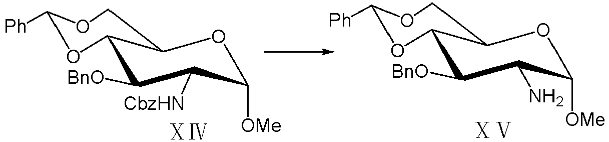

300 g compound of Formula XIV was dissolved in 3 L ethylene glycol monomethyl ether, followed by the addition of 400 g potassium hydroxide. Subsequently, it was heated up to 100°C for reaction. After the reaction was finished, most of the solvent was removed under reduced pressure. It was then poured into 4 L water slowly, and filtered to collect the solid. The crude product was dissolved in ethyl acetate, washed by water, dried, concentrated under reduced pressure, and recrystallized to give 210 g product as brown solid. The structure was characterized with a small amount of product after purification by column chromatography.

The MS, and ¹H NMR data for the compound of Formula XV: ESI-MS(m/z): 372[M+H]⁺; ¹H NMR(400 MHz, CDCl₃): δ 7.52-7.50(m, 2 H), 7.41-7.30(m, 8 H), 5.60(s, 1 H), 5.02(d, J = 11.2 Hz, 1 H), 4.76(d, J = 4.0 Hz, 1 H), 4.70(d, J = 11.2 Hz, 1 H), 4.30(dd, J₁ = 9.6 Hz, J2 = 4.4 Hz, 1 H), 3.87-3.76(m, 2 H), 3.65(m, 2 H), 3.41(s, 3 H), 2.86(dd, J₁ = 8.8 Hz, J₂ = 3.6 Hz, 1 H).

### Example 9: Preparation of methyl 2-azido-3-O-benzyl-4,6-O-benzylidene-2-deoxy-α-D-glucopyranoside (XVI)

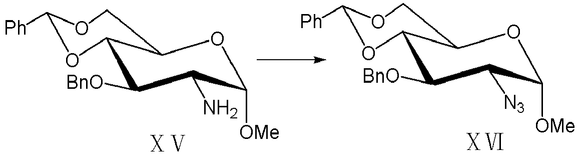

37.2 g compound of Formula XV was dissolved in 500 ml methanol, into which 0.4 g chalcanthite and 27.6 g potassium carbonate were added. Subsequently, 37 g 1H-imidazole-1-sulfonyl azide hydrochloride was added batchwise while cooled in an ice water bath. After the addition, the ice water bath was removed, and it was stirred at room temperature until the reaction was finished. It was then concentrated under reduced pressure, and the resultant solution was diluted by water. The pH was adjusted to weak acid by dilute hydrochloric acid in an ice bath. Subsequently, it was extracted by ethyl acetate, dried, filtered, and concentrated to give a crude product. It was pulped in methanol, and filtered to collect the solid. Finally, 26 g white solid was obtained. The structure was characterized with a small amount of product after purification by column chromatography.

The MS, and ¹H NMR data for the compound of Formula XVI: ESI-MS(m/z): 398[M+H]⁺; ¹H NMR(400 MHz, CDCl₃): δ 7.52-7.29(m, 10 H), 5.61(s, 1 H), 4.96(d, J = 11.2 Hz, 1 H), 4.82(d, J = 11.2 Hz, 1 H), 4.80(s, 1 H), 4.32(dd, J₁ = 10.0 Hz, J₂ = 4.8 Hz, 1 H), 4.07(d, J = 9.2 Hz, 1 H), 3.88(dd, J₁ = 10.0 Hz, J₂ = 4.8 Hz, 1 H), 3.81-3.70(m, 2 H), 3.47-3.44(m, 1 H), 3.46(s, 3 H).

### Example 10: the preparation of methyl 2-azido-3-O-benzyl-4,6-O-benzylidene-2-deoxy-α-D-glucopyranoside (XVI)

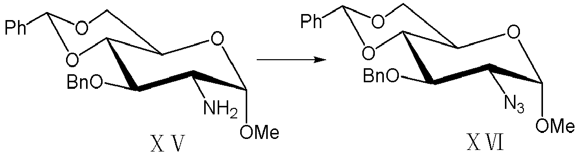

18.0 g compound of Formula XV was dissolved in 200 ml methanol and 10 ml water, into which 0.2 g chalcanthite and 13.0 g potassium carbonate were added. Subsequently, 120 ml trifluoromethanesulfonyl azide solution in dichloromethane (1 mol/L) was added dropwise while cooled in an ice water bath. After the addition, the ice water bath was removed, and it was stirred at room temperature until the reaction was finished. It was concentrated under reduced pressure, and the resultant solution was diluted by water. Subsequently, it was extracted by ethyl acetate, dried, filtered, and concentrated to give a crude product. It was pulped in methanol, and filtered to collect the solid. Finally, 10.4 g white solid was obtained. The structure was characterized with a small amount of product after purification by column chromatography.

The MS, and ¹H NMR data for the compound of Formula XVI: ESI-MS(m/z): 398[M+H]⁺; ¹H NMR(400 MHz, CDCl₃): δ 7.52-7.29(m, 10 H), 5.61(s, 1 H), 4.96(d, J = 11.2 Hz, 1 H), 4.82(d, J = 11.2 Hz, 1 H), 4.80(s, 1 H), 4.32(dd, J₁ = 10.0 Hz, J₂ = 4.8 Hz, 1 H), 4.07(d, J = 9.2 Hz, 1 H), 3.88(dd, J₁ = 10.0 Hz, J₂ = 4.8 Hz, 1 H), 3.81-3.70(m, 2 H), 3.47-3.44(m, 1 H), 3.46(s, 3 H).

### Example 11: Preparation of methyl 2-azido-3-O-benzyl-2-deoxy-α-D-glucopyranoside (XVII)

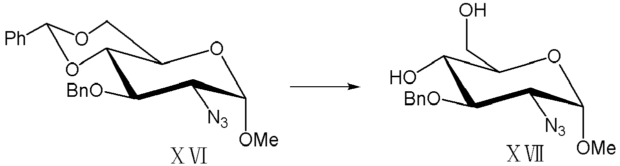

50 g compound of Formula XVI was dissolved in 700 ml dichloromethane, followed by the addition of 15 ml water. Subsequently, 75 ml trifluoroacetic acid was added dropwise into the reaction system which was placed in an ice water bath and stirred until the reaction was finished. The pH value was maintained in the range from 11 to 13 by adding 30% NaOH solution dropwise into the reaction system placed in an ice water bath. It was then extracted twice by dichloromethane, and the organic phase was dried over anhydrous sodium sulfate, and filtered. The filtrate was concentrated and recrystallized to give 33 g product.

The MS, and ¹H NMR data for the compound of Formula XVII: ESI-MS(m/z): 282[M-N₂+H]⁺; ¹H NMR(400 MHz, CDCl₃): δ 7.42-7.30(m, 5 H), 4.94(d, J = 10.8 Hz, 1 H), 4.79(d, J = 11.2 Hz, 1 H), 4.77(s, 1 H), 3.84-3.79(m, 3 H), 3.67-3.63(m, 2 H), 3.42(s, 3 H), 3.33(dd, J₁ = 10.0 Hz, J₂ = 3.2 Hz, 1 H), 3.10(br, 1 H), 2.49(br, 1 H).

### Examples 12: Preparation of methyl 2-azido-6-O-benzoyl-3-O-benzyl-2-deoxy-α-D-glucopyranoside (XII)

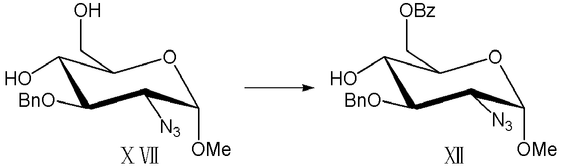

In a 3-neck flask, 50 g compound of Formula XVII was dissolved in 400 ml dichloromethane, into which 70 ml pyridine was added. Subsequently, when cooled in an ice water bath, 21 ml benzoyl chloride was added dropwise. After the addition, the reaction temperature increased spontaneously. The reaction was stopped by quenching with water, and the system stood for phase separation. The water phase was extracted by dichloromethane. The organic phases were combined, washed sequentially by saturated NaHCO₃ solution and 2% HCl solution. Subsequently, it was dried over anhydrous sodium sulfate, and filtered. The filtrate was concentrated and recrystallized to give 57 g product.

The MS, and ¹H NMR data for the compound of Formula XII: ESI-MS(m/z): 386[M-N₂+H]⁺; ¹H NMR(400 MHz, CDCl₃): δ 8.03(d, J = 8.0 Hz, 2 H), 7.58-7.28(m, 8 H), 4.92(d, J = 10.8 Hz, 1 H), 4.84-4.80(m, 2 H), 4.71(dd, J₁ = 12 Hz, J₂ = 4.0 Hz, 1 H), 4.48(d, J = 12 Hz, 1 H), 3.89-3.82(m, 2 H), 3.57(td, J₁ = 9.2 Hz, J₂ = 3.6 Hz, 1 H), 3.44(s, 3 H), 3.36(dd, J₁ = 10.0 Hz, J₂ = 3.2 Hz, 1 H), 2.90(d, J = 3.6 Hz, 1 H).

### Example 13: Preparation of methyl O-(4,6-di-O-acetyl-2-O-benzoyl-3-O-benzyl-α-L-idopyranosyl)-(1→4)-2-azido-6-O-benzoyl-3-O-benzyl-2-deoxy-α-D-glucopyranoside (VII)

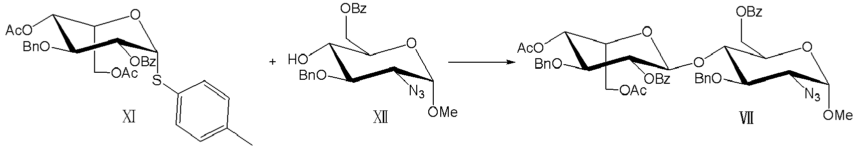

102 g compound of Formula XI, 69 g compound of Formula XII and 100 g 4Å molecular sieve were dissolved in 1.3 L dichloromethane under nitrogen atmosphere. After the reaction solution was cooled to -20°C, 67 g N-iodosuccinimide and 4.42 g trifluoromethanesulfonic acid were added, and the mixture was stirred until the reaction was finished. The reaction was quenched by adding saturated sodium bicarbonate solution, and the mixture stood for phase separation. The organic phase was washed by saturated sodium chloride solution, dried and concentrated. 133 g solid was obtained by purification using column chromatography.

The MS, and ¹H NMR data for the compound of Formula VII: ESI-MS(m/z): 876[M+Na]⁺; ¹H NMR(400 MHz, CDCl₃): δ 8.04(d, J = 7.6 Hz, 2 H), 7.97(d, J = 7.2 Hz, 2 H), 7.59-7.20(m, 16 H), 5.27(s, 1 H), 5.18(s, 1 H), 4.90-4.64(m, 8), 4.48(dd, J₁ = 12.4 Hz, J₂ = 3.2 Hz, 1 H), 4.06-3.84(m, 6 H), 3.45-3.41(m, 4 H), 1.98(s, 3 H), 1.91(s, 3 H).

### Example 14: Preparation of methyl O-(4,6-di-O-acetyl-2-O-benzoyl-3-O-benzyl-α-L-idopyranosyl)-(1→4)-2-azido-6-O-benzoyl-3-O-benzyl-2-deoxy-α-D-glucopyranoside (VII)

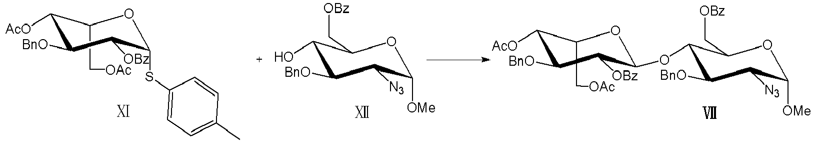

27.0 g compound of Formula XI, 18.3 g compound of Formula XII and 25 g 4Å molecular sieve were dissolved in 0.3 L dichloromethane under nitrogen atmosphere. After the reaction solution was cooled to -5°C, 14.8 g N-iodosuccinimide and 1.13 g silver trifluoromethanesulfonate were added, and the mixture was stirred until the reaction was finished. The reaction was quenched by adding saturated sodium bicarbonate solution, and the mixture stood for phase separation. The organic phase was washed by saturated sodium chloride solution, dried and concentrated. 29.3 g solid was obtained by purification using column chromatography.

The MS, and ¹H NMR data for the compound of Formula VII: ESI-MS(m/z): 876[M+Na]⁺; ¹H NMR(400 MHz, CDCl₃): δ 8.04(d, J = 7.6 Hz, 2 H), 7.97(d, J = 7.2 Hz, 2 H), 7.59-7.20(m, 16 H), 5.27(s, 1 H), 5.18(s, 1 H), 4.90-4.64(m, 8), 4.48(dd, J₁ = 12.4 Hz, J₂ = 3.2 Hz, 1 H), 4.06-3.84(m, 6 H), 3.45-3.41(m, 4 H), 1.98(s, 3 H), 1.91(s, 3 H).

### Example 15: Preparation of methyl O-(2-O-benzoyl-3-O-benzyl-α-L-idopyranosyl)-(1→4)-2-azido-6-O-benzoyl-3-O-benzyl-2-d eoxy-α-D-glucopyranoside (VIII)

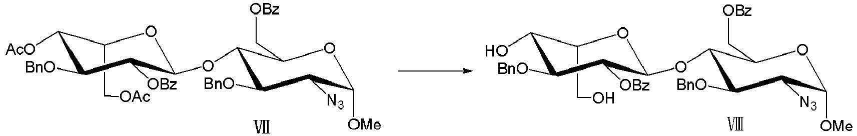

46.7 g compound of Formula VII was dissolved in 500 ml 0.2 M hydrogen chloride solution in methanol. The mixture was stirred at room temperature until the reaction was finished. The reaction was quenched by adding triethylamine in an ice water bath. After the reaction solution was concentrated under reduced pressure, it was dissolved in ethyl acetate, and washed sequentially with water and saturated sodium chloride aqueous solution. Subsequently, it was dried over anhydrous sodium sulfate, and concentrated. 37 g oil product was obtained by purification using column chromatography.

The MS, and ¹H NMR data for the compound of Formula VIII: ESI-MS(m/z): 792[M+Na]⁺; ¹H NMR(400 MHz, CDCl₃): δ 8.00(d, J = 7.2 Hz, 2 H), 7.94(d, J = 7.2 Hz, 2 H), 7.57-7.25(m, 16 H), 5.22(s, 1 H), 5.13(s, 1 H), 4.90-4.79(m, 4 H), 4.68-4.63(m, 2 H), 4.49(dd, J₁ = 12.4 Hz, J₂ = 3.2 Hz, 1 H), 4.28(t, J = 4.8 Hz, 1 H), 4.06-4.02(m, 2 H), 3.91-3.83(m, 2 H), 3.67(s, 1 H), 3.52(dd, J₁ = 10.0 Hz, J₂ = 3.6 Hz, 1 H), 3.47(s, 3 H), 3.33(dd, J₁ = 12.0 Hz, J₂ = 6.4 Hz, 1 H), 3.20(dd, J₁ = 12.0 Hz, J₂ = 4.8 Hz, 1 H).

### Example 16: Preparation of methyl O-(2-O-benzoyl-3-O-benzyl-α-L-idopyranuronosyl)-(1→4)- 2-azido-6-O-benzoyl-3-O-benzyl -2-deoxy-α-D-glucopyranoside (IX)

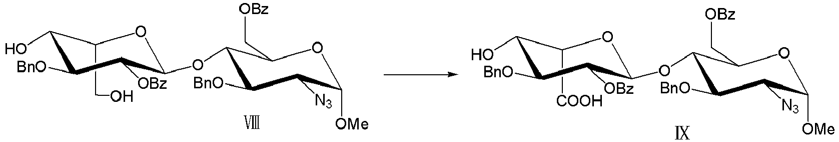

20 g compound of Formula VIII was dissolved in a mixture of 150 ml dichloromethane and 50 ml water, and cooled in an water bath. 20 g iodobenzene diacetate and 0.78 g 2,2,6,6-tetramethyl-1-piperidinyloxy were added, and stirred until the reaction was finished. 20 ml saturated sodium thiosulfate solution was added, and stirred for 30 min. The mixture stood for phase separation, and the water phase was extracted by dichloromethane. The organic phases were combined, washed with saturated sodium chloride solution, dried over anhydrous sodium sulfate, and concentrated. 31 g crude oil product was obtained, which was used directly in the next step of reaction with no further purification.

The MS data for the compound of Formula IX: ESI-MS(m/z): 806[M+Na]⁺.

### Example 17: Preparation of methyl O-(2-O-benzoyl-3-O-benzyl-α-L-idopyranuronosyl)-(1→4)-2-azido-6-O-benzoyl-3-O-benzyl -2-deoxy-α-D-glucopyranoside (IX)

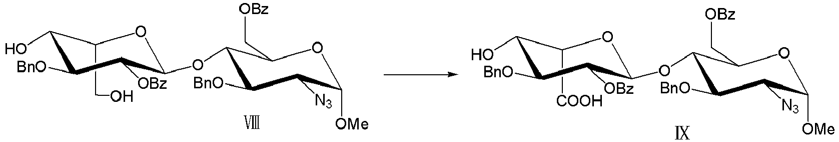

11.2 g compound of Formula VIII was dissolved in a mixture of 25 ml dichloromethane and 25 ml saturated sodium bicarbonate-sodium carbonate aqueous solution, and cooled in a water bath. 0.69 g potassium bromide, 0.33 g tetrabutyl ammonium bromide and 0.04 g 2,2,6,6-tetramethyl-1-piperidinyloxy were sequentially added, and 5.0 g calcium hypochlorite was added batchwise. The mixture was stirred until the reaction was finished. 20 ml saturated sodium thiosulfate solution was added and stirred for 30 min. The mixture stood for phase separation, and the water phase was extracted by dichloromethane. The organic phases were combined, washed with saturated sodium chloride solution, dried over anhydrous sodium sulfate, and concentrated. 7.8 g crude oil product was obtained, which was used directly in the next step of reaction with no further purification.

The MS data for the compound of Formula IX: ESI-MS(m/z): 806[M+Na]⁺.

### Example 18: Preparation of methyl O-(methyl 2-O-benzoyl-3-O-benzyl-α-L-idopyranosyluronate)-(1→4)-2-azido-6-O-benzoyl-3-O-benzyl-2-deoxy-α-D-glucopyranoside (X)

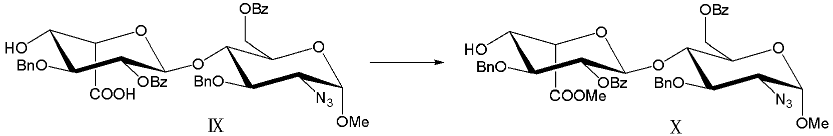

31 g crude product of compound of Formula IX (obtained in Example 16) and 17.75 g iodomethane were dissolved in acetonitrile, into which 12.5 g potassium bicarbonate was added, and stirred until the reaction was finished. After the reaction solution was concentrated under reduced pressure, it was dissolved in ethyl acetate, and washed sequentially with water and saturated sodium chloride solution. Subsequently, it was dried over anhydrous sodium sulfate, and concentrated. 17 g solid was obtained by purification using column chromatography.

The MS, and ¹H NMR data for the compound of Formula X: ESI-MS(m/z): 770[M-N₂+H]⁺; ¹H NMR(400 MHz, CDCl₃): δ 8.04(d, J = 7.2 Hz, 2 H), 7.92(d, J = 7.2 Hz, 2 H), 7.57-7.23(m, 16 H), 5.37(s, 1 H), 5.21(s, 1 H), 4.97(d, J = 2.4 Hz, 1 H), 4.84-4.77(m, 4 H), 4.73(d, J = 5.6 Hz, 1 H), 4.69(d, J = 6.4 Hz, 1 H), 4.49(dd, J₁ = 12.4 Hz, J₂ = 4.0 Hz, 1 H), 4.06-4.00(m, 3 H), 3.92-3.86(m, 2 H), 3.49-3.46(m, 1 H), 3.46(s, 3 H), 3.44(s, 3 H), 2.66(d, J = 10.8 Hz, 1 H).

### Example 19: Preparation of methyl O-(methyl 2-O-benzoyl-3-O-benzyl-α-L-idopyranosyluronate)-(1→4)-2-azido-6-O-benzoyl-3-O-benzyl-2-deoxy-α-D-glucopyranoside (X)

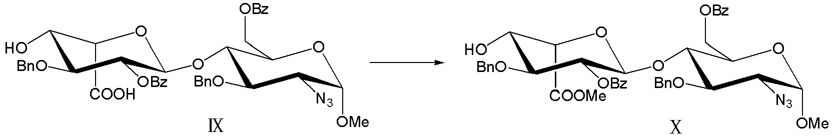

7.8 g crude product of compound of Formula IX (obtained in Example 17) was dissolved in 50 ml ether, into which 25 ml 1 mol/L diazomethane-ether solution was slowly added dropwise, and the mixture was stirred until the reaction was finished. 1.4 g acetic acid was added and stirred for 2 h. After the reaction solution was concentrated under reduced pressure, it was dissolved in ethyl acetate, and washed sequentially with saturated aqueous sodium bicarbonate solution and saturated sodium chloride solution. Subsequently, it was dried over anhydrous sodium sulfate, and concentrated. 6.3 g solid was obtained by purification using column chromatography.

The MS, and ¹H NMR data for the compound of Formula X: ESI-MS(m/z): 770[M-N₂+H]⁺; ¹H NMR(400 MHz, CDCl₃): δ 8.04(d, J = 7.2 Hz, 2 H), 7.92(d, J = 7.2 Hz, 2 H), 7.57-7.23(m, 16 H), 5.37(s, 1 H), 5.21(s, 1 H), 4.97(d, J = 2.4 Hz, 1 H), 4.84-4.77(m, 4 H), 4.73(d, J = 5.6 Hz, 1 H), 4.69(d, J = 6.4 Hz, 1 H), 4.49(dd, J₁ = 12.4 Hz, J₂ = 4.0 Hz, 1 H), 4.06-4.00(m, 3 H), 3.92-3.86(m, 2 H), 3.49-3.46(m, 1 H), 3.46(s, 3 H), 3.44(s, 3 H), 2.66(d, J = 10.8 Hz, 1 H).

### Example 20: Preparation of methyl O-(6-O-acetyl-2-azido-3,4-di-O-benzyl-2-deoxy-α-D-glucopyranosyl)-(1→4)-O-(methyl 2,3-di-O-benzyl-β-D-glucopyranosyluronate)-(1→4)-O-(3,6-di-O-acetyl-2-azido-2-deoxy-α-D-glucopyranosyl)-(1→4)-O-(methyl 2-O-benzoyl-3-O-benzyl-α-L-idopyranosyluronate)-(1→4)-2-azido-6-O-benzoyl-3-O-benzyl-2-deoxy-α-D-glucopyranoside (XVIII)

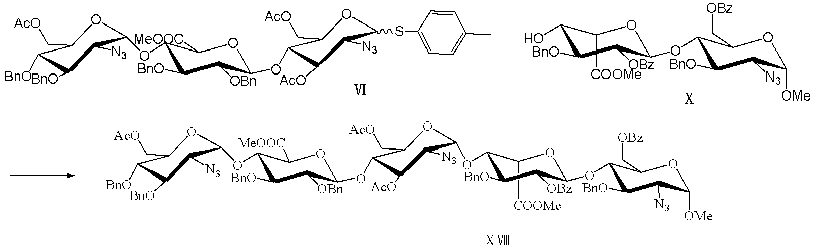

13 g compound of Formula VI, 8.8 g compound of Formula X and 13 g 4Å molecular sieve were dissolved in 200 mL dichloromethane under nitrogen atomsphere. After the reaction solution was cooled to -20°C, 4.48 g N-iodosuccinimide and 0.9 g trifluoromethanesulfonic acid were added, and the mixture was stirred until the reaction was finished. The reaction was quenched by adding saturated sodium bicarbonate solution, and the mixture stood for phase separation. The organic phase was washed by saturated sodium chloride solution, dried and concentrated. 16.0 g solid was obtained by purification using column chromatography.

The MS, and ¹H NMR data for the compound of Formula XVIII: ESI-MS(m/z): 1820[M-N₂+H]⁺, 1865[M+NH₄]⁺; ¹H NMR(400 MHz, CDCl₃): δ 8.10(d, J = 7.2 Hz, 2 H), 8.01(d, J = 7.2 Hz, 2 H), 7.53-7.17(m, 36 H), 5.73(d, J = 6.0 Hz, 1 H), 5.50(d, J = 3.6 Hz, 1 H), 5.35-5.26(m, 2 H), 5.11(d, J = 3.6 Hz, 1 H), 4.98(d, J = 4.0 Hz, 1 H), 4.96(d, J = 4.0 Hz, 1 H), 4.86-4.53(m, 13 H), 4.47-4.42(m, 2 H), 4.35(d, J = 7.6 Hz, 1 H), 4.28-4.12(m, 5 H), 4.06-3.97(m, 3 H), 3.92-3.80(m, 4 H), 3.74(s, 3 H), 3.72-3.66(m, 2 H), 3.54(s, 3 H), 3.50-3.49(m, 2 H), 3.44-3.39(m, 2 H), 3.35(s, 3 H), 3.28-3.19(m, 2 H).

A person of ordinary skill in the art is capable of implementing or using the present invention based on the above description of the Examples disclosed. It is apparent to a person of ordinary skill in the art that various modifications can be made to these Examples. The general principles defined in the invention can be achieved in other Examples without departing from the spirit and scope of the invention. Therefore, the present invention will be applied to the widest scope consistent with the principles and novel features disclosed herein, rather than being limited to the Examples described herein.

## Claims

1. A compound of Formula V, 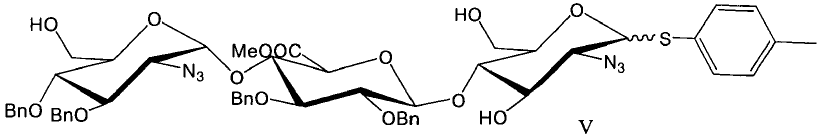 wherein, the configuration of the monosaccharide unit and the stereochemistry of the linkages among each monosaccharides are expressed as D-glucose-α-1,4-D-glucuronic acid-β-1,4-D-glucose.

2. A compound of Formula VI, 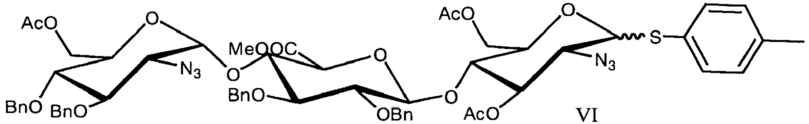 wherein, the configuration of the monosaccharide unit and the stereochemistry of the linkages among each monosaccharides are expressed as D-glucose-α-1,4-D-glucuronic acid-β-1,4-D-glucose.

3. A method for preparing the compound of formula V, which is **characterized in that**: the compound of formula V is obtained by removing three p-methoxybenzyl groups from the compound of formula IV:

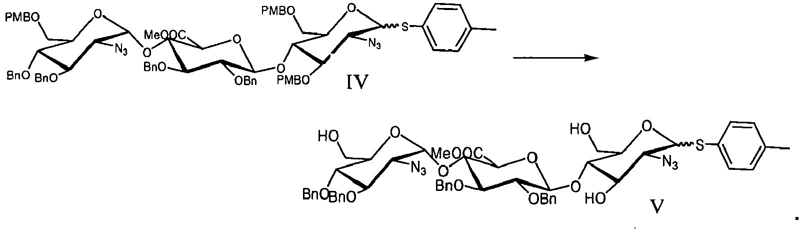

4. A method for preparing the compound of formula VI, which is **characterized in that**: the compound of Formula VI is obtained by reacting the compound of formula V with an acetylation reagent under alkaline condition in order to protect the three exposed hydroxy groups with the acetyl group: 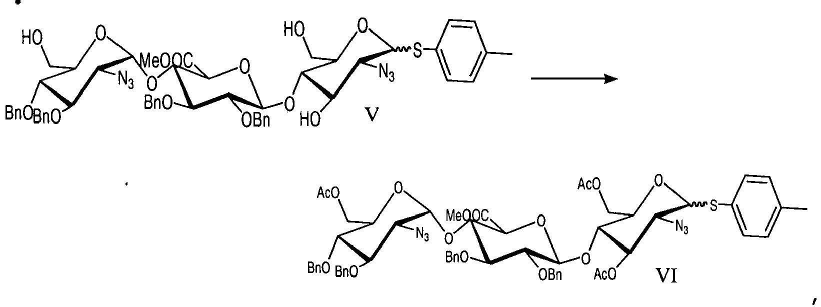 preferably, the base is selected from an organic base or an inorganic base, wherein the organic base is selected from pyridine or triethylamine, and the inorganic base is selected from potassium carbonate, sodium carbonate or sodium bicarbonate , the acetylation reagent is selected from acetic anhydride or acetyl chloride.

5. A method for preparing the compound of Formula XVIII, which is **characterized in that**: the compound of Formula XVIII, a fully protected pentasaccharide, is obtained by the glycosylation reaction of the compound of Formula VI and the compound of Formula X under the condition that allows the formation of glucosidic linkage:

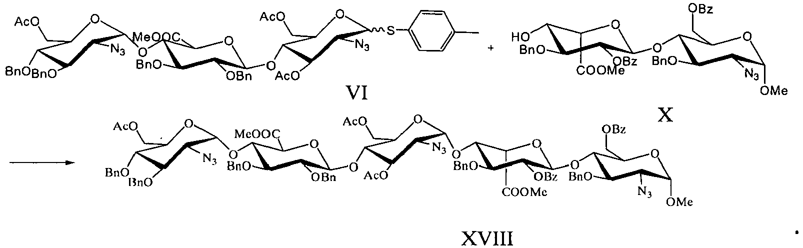

6. The method according to claim 5, wherein the compound of Formula X is obtained by the methyl esterification of the compound of Formula IX using a methylation reagent: 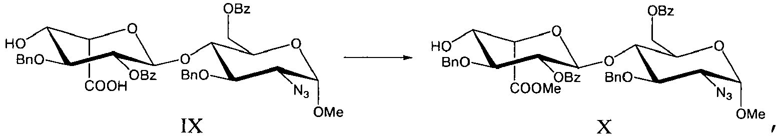 wherein the methylation reagent is preferably selected from diazomethane, (trimethylsilyl)diazomethane, iodomethane, bromomethane or chloromethane.

7. The method according to claim 5, which is **characterized in that**: the compound of Formula IX is obtained by converting the exposed primary alcoholic hydroxyl group in the compound of Formula VIII into carboxyl via oxidation in the presence of oxidizing agent and catalyst: 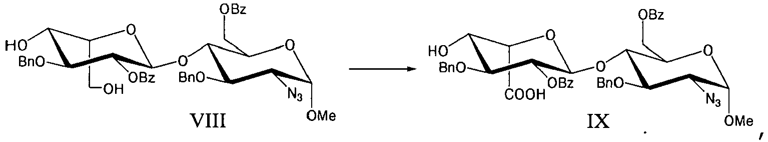 preferably, the oxidizing agent is selected from iodobenzene diacetate, calcium hypochlorite or sodium hypochlorite, and the catalyst is 2,2,6,6-tetramethyl-1-piperidinyloxy.

8. The method according to claim 7, which is **characterized in that**: the compound of Formula VIII is obtained by removing the acetyl-protecting group of the compound of Formula VII in the presence of a deacetylation reagent: 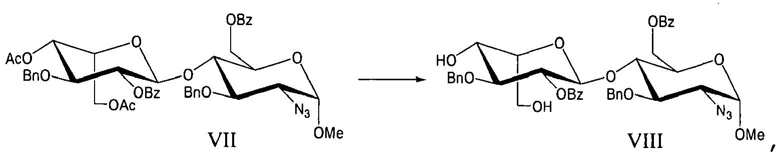 preferably, the deacetylation reagent is selected from a solution of hydrogen chloride in methanol, a solution of hydrogen chloride in ethanol, a solution of triethylamine in methanol, a solution of triethylamine in ethanol, sodium methoxide or sodium ethoxide.

9. The method according to claim 8, which is **characterized in that**: the compound of Formula VII is obtained by the glycosylation reaction of the compound of Formula XI and the compound of Formula XII under the condition that allows the formation of glucosidic linkage:

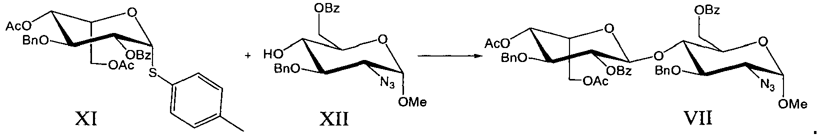

10. The method according to claim 9, which is **characterized in that**: the compound of Formula XI is obtained by reacting the compound of formula XIII with an acetylation reagent under alkaline condition in order to protect the 2 exposed hydroxy groups with the acetyl group: 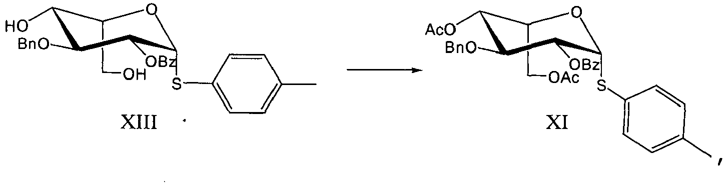 preferably, the base is selected from an organic base or an inorganic base, wherein the organic base is selected from pyridine or triethylamine, and the inorganic base is selected from potassium carbonate, sodium carbonate or sodium bicarbonate; the acetylation reagent is selected from acetic anhydride or acetyl chloride.

11. The method according to claim 9, which is **characterized in that**: the preparing method for the compound of Formula XII comprises the following steps:
1) the compound of Formula XIV is treated by a strong base in order to give the compound of Formula XV by removing the carbobenzoxy group (-Cbz), and exposing the amino group: 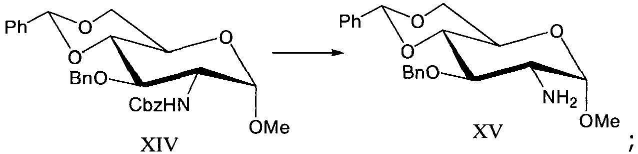
2) the compound of Formula XV is reacted with a diazo transfer reagent in order to give the compound of Formula XVI by converting the exposed amino groups in the compound of Formula XV into the azido group: 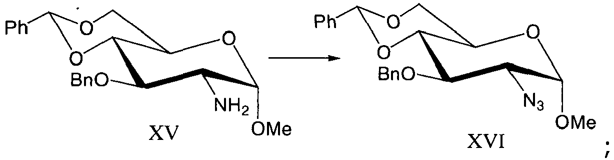
3) the benzylidene is removed from the compound of Formula XVI under acidic condition to give the compound of Formula XVII: 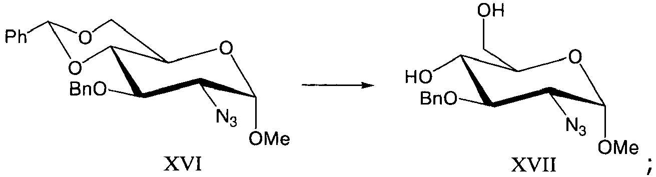
4) the compound of Formula XVII is reacted with a benzoylation reagent under alkaline condition to give the compound of Formula XII by protecting the primary alcoholic hydroxyl group with the benzoyl group:

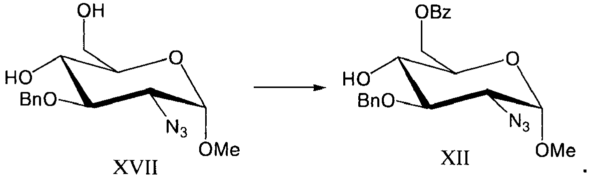

## Patentansprüche

1. Verbindung der Formel V 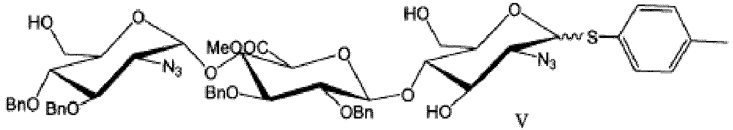 wobei die Konfiguration der Monosaccharideinheit und die Stereochemie der Bindungen unter den Monosacchariden jeweils als D-Glucose-α-1,4-D-glucuronsäure-β-1,4-D-glucose ausgedrückt wird.

2. Verbindung der Formel VI 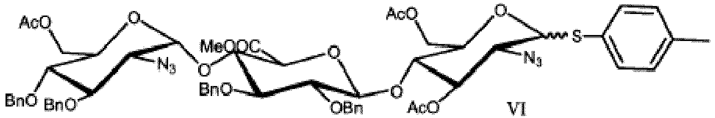 wobei die Konfiguration der Monosaccharideinheit und die Stereochemie der Bindungen unter den Monosacchariden jeweils als D-Glucose-α-1,4-D-glucuronsäure-β-1,4-D-glucose ausgedrückt wird.

3. Verfahren zur Herstellung der Verbindung der Formel V, das **dadurch gekennzeichnet ist, dass** die Verbindung der Formel V durch Entfernen von drei p-Methoxybenzylgruppen von der Verbindung der Formel IV erhalten wird:

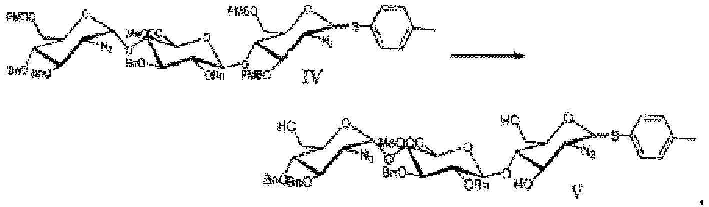

4. Verfahren zur Herstellung der Verbindung der Formel VI, das **dadurch gekennzeichnet ist, dass** die Verbindung der Formel VI durch Umsetzen der Verbindung der Formel V mit einem Acetylierungsreagens unter alkalischen Bedingungen, um die drei exponierten Hydroxygruppen mit der Acetylgruppe zu schützen, erhalten wird: 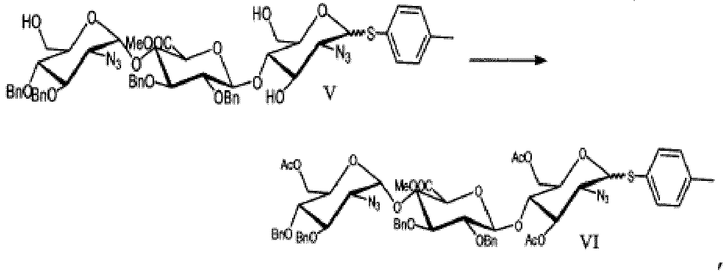 wobei die Base vorzugsweise aus einer organischen Base oder einer anorganischen Base ausgewählt ist, wobei die organische Base aus Pyridin oder Triethylamin ausgewählt ist und die anorganische Base aus Kaliumcarbonat, Natriumcarbonat oder Natriumhydrogencarbonat ausgewählt ist, das Acetylierungsreagens aus Essigsäureanhydrid oder Acetylchlorid ausgewählt ist.

5. Verfahren zur Herstellung der Verbindung der Formel XVIII, das **dadurch gekennzeichnet ist, dass** die Verbindung der Formel XVIII, ein vollständig geschütztes Pentasaccharid, durch die Glycosylierungsreaktion der Verbindung der Formel VI und der Verbindung der Formel X unter Bedingungen, die die Bildung einer glucosidischen Bindung ermöglichen, erhalten wird:

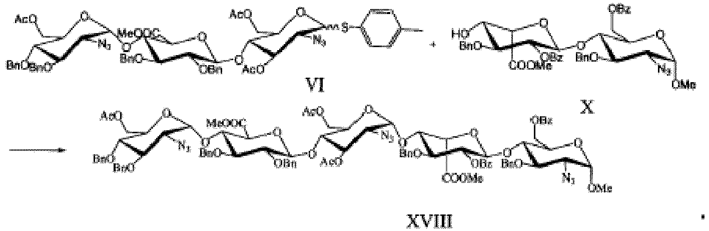

6. Verfahren gemäß Anspruch 5, wobei die Verbindung der Formel X durch die Methylveresterung der Verbindung der Formel IX unter Verwendung eines Methylierungsreagens erhalten wird: 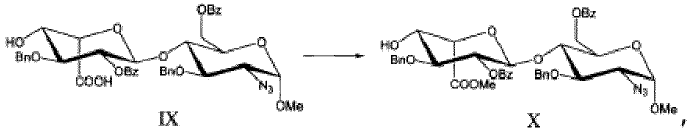 wobei das Methylierungsreagens vorzugsweise aus Diazomethan, (Trimethylsilyl)diazomethan, Iodmethan, Brommethan oder Chlormethan ausgewählt ist.

7. Verfahren gemäß Anspruch 5, das **dadurch gekennzeichnet ist, dass** die Verbindung der Formel IX durch Umsetzen der exponierten primären alkoholischen Hydroxygruppe in der Verbindung der Formel VIII zu einer Carboxygruppe über eine Oxidation in Gegenwart von Oxidationsmittel und Katalysator erhalten wird: 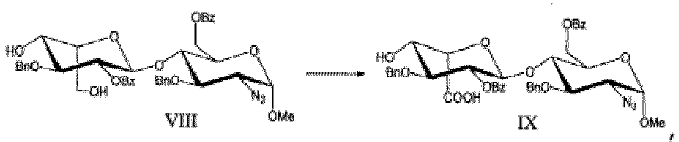 wobei vorzugsweise das Oxidationsmittel aus Iodbenzoldiacetat, Calciumhypochlorit oder Natriumhypochlorit ausgewählt ist und es sich bei dem Katalysator um 2,2,6,6-Tetramethyl-1-piperidinyloxy handelt.

8. Verfahren gemäß Anspruch 7, das **dadurch gekennzeichnet ist, dass** die Verbindung der Formel VIII durch Entfernen der Acetyl-Schutzgruppe der Verbindung der Formel VII in Gegenwart eines Deacetylierungsreagens erhalten wird: 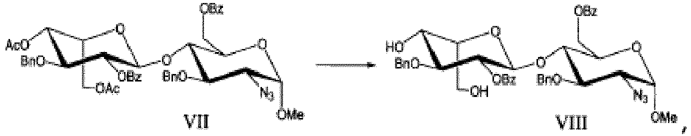 wobei vorzugsweise das Deacetylierungsreagens aus einer Lösung von Chlorwasserstoff in Methanol, einer Lösung von Chlorwasserstoff in Ethanol, einer Lösung von Triethylamin in Methanol, einer Lösung von Triethylamin in Ethanol, Natriummethoxid oder Natriumethoxid ausgewählt ist.

9. Verfahren gemäß Anspruch 8, das **dadurch gekennzeichnet ist, dass** die Verbindung der Formel VII durch die Glycosylierungsreaktion der Verbindung der Formel XI und der Verbindung der Formel XII unter Bedingungen, die die Bildung einer glucosidischen Bindung ermöglichen, erhalten wird:

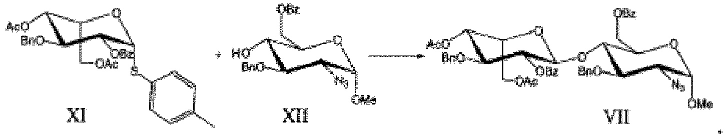

10. Verfahren gemäß Anspruch 9, das **dadurch gekennzeichnet ist, dass** die Verbindung der Formel XI durch Umsetzen der Verbindung der Formel XIII mit einem Acetylierungsreagens unter alkalischen Bedingungen, um die 2 exponierten Hydroxygruppen mit der Acetylgruppe zu schützen, erhalten wird: 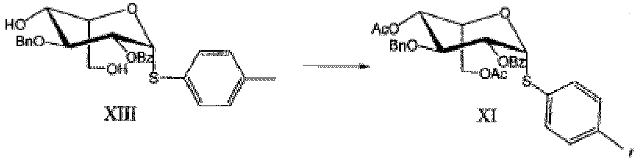 wobei die Base vorzugsweise aus einer organischen Base oder einer anorganischen Base ausgewählt ist, wobei die organische Base aus Pyridin oder Triethylamin ausgewählt ist und die anorganische Base aus Kaliumcarbonat, Natriumcarbonat oder Natriumhydrogencarbonat ausgewählt ist, das Acetylierungsreagens aus Essigsäureanhydrid oder Acetylchlorid ausgewählt ist.

11. Verfahren gemäß Anspruch 9, das **dadurch gekennzeichnet ist, dass** das Herstellungsverfahren für die Verbindung der Formel XII die folgenden Schritte umfasst:
1) die Verbindung der Formel XIV wird mit einer starken Base behandelt, um die Verbindung der Formel XV zu erhalten, indem man die Carbobenzoxygruppe (-Cbz) entfernt und die Aminogruppe exponiert: 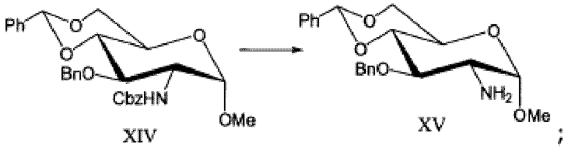
2) die Verbindung der Formel XV wird mit einem Diazo-Übertragungsreagens umgesetzt, um die Verbindung der Formel XVI zu erhalten, indem man die exponierten Aminogruppen in der Verbindung der Formel XV zu der Azidogruppe umsetzt: 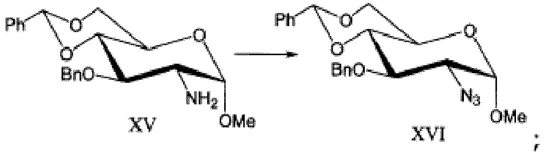
3) die Benzylidengruppe wird unter sauren Bedingungen aus der Verbindung der Formel XVI entfernt, was die Verbindung der Formel XVII ergibt: 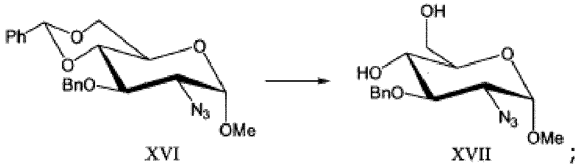
4) die Verbindung der Formel XVII wird unter alkalischen Bedingungen mit einem Benzoylierungsreagens umgesetzt, was die Verbindung der Formel XII ergibt, indem man die primären alkoholischen Hydroxygruppen mit der Benzoylgruppe schützt:

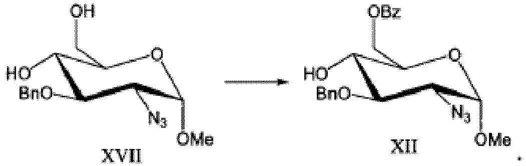

## Revendications

1. Composé de formule V, 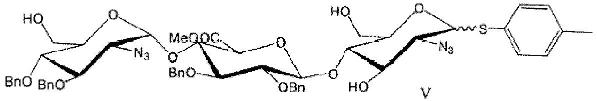 dans lequel, la configuration du motif monosaccharide et la stéréochimie des liaisons parmi chaque monosaccharide sont exprimées sous la forme D-glucose-α-1,4-acide D-glucuronique-β-1,4-D-glucose.

2. Composé de formule VI, 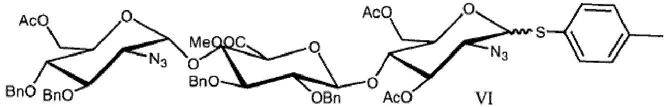 dans lequel, la configuration du motif monosaccharide et la stéréochimie des liaisons parmi chaque monosaccharide sont exprimées sous la forme D-glucose-α-1,4-acide D-glucuronique-β-1,4-D-glucose.

3. Procédé de préparation du composé de formule V, qui est **caractérisé en ce que** : le composé de formule V est obtenu en éliminant trois groupes p-méthoxybenzyles du composé de formule IV :

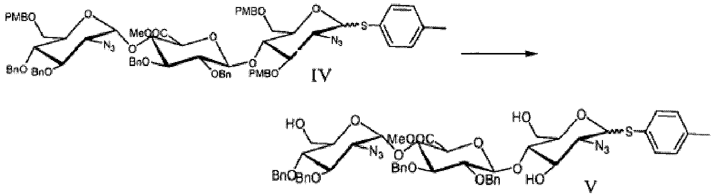

4. Procédé de préparation du composé de formule VI, qui est **caractérisé en ce que** : le composé de Formule VI est obtenu en faisant réagir le composé de formule V avec un réactif d'acétylation en condition alcaline afin de protéger les trois groupes hydroxy exposés avec le groupe acétyle : 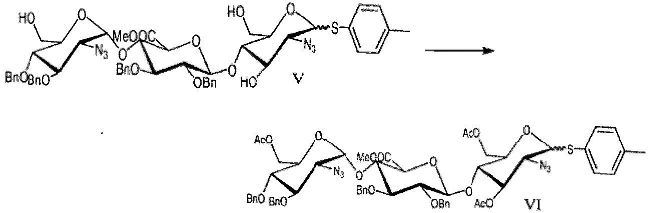 de préférence, la base est choisie parmi une base organique ou une base inorganique, dans lequel la base organique est choisie parmi la pyridine ou la triéthylamine, et la base inorganique est choisie parmi le carbonate de potassium, le carbonate de sodium ou le bicarbonate de sodium, le réactif d'acétylation est choisi parmi l'anhydride acétique ou le chlorure d'acétyle.

5. Procédé de préparation du composé de Formule XVIII, qui est **caractérisé en ce que** : le composé de Formule XVIII, un pentasaccharide pleinement protégé, est obtenu par la réaction de glycosylation du composé de Formule VI et du composé de Formule X dans la condition qui permet la formation de liaison glucosique :

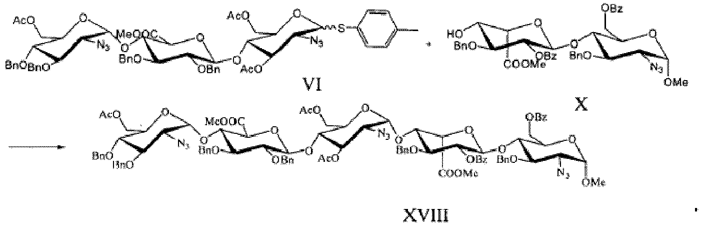

6. Procédé selon la revendication 5, dans lequel le composé de Formule X est obtenu par la méthyle estérification du composé de Formule IX à l'aide d'un réactif de méthylation : 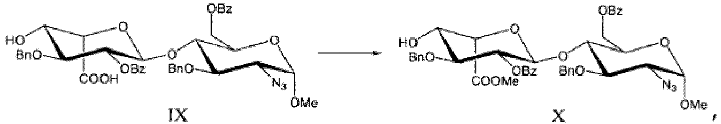 dans lequel le réactif de méthylation est de préférence choisi parmi le diazométhane, le (triméthylsilyl)diazométhane, l'iodométhane, le bromométhane ou le chlorométhane.

7. Procédé selon la revendication 5, qui est **caractérisé en ce que** : le composé de Formule IX est obtenu en convertissant le groupe hydroxyle alcoolique primaire exposé dans le composé de Formule VIII en carboxyle via une oxydation en présence d'un agent oxydant et d'un catalyseur : 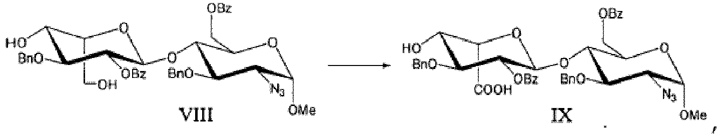 de préférence, l'agent oxydant est choisi parmi le diacétate d'iodobenzène, l'hypochlorite de calcium ou l'hypochlorite de sodium, et le catalyseur est le 2,2,6,6-tétraméthyl-1-pipéridinyloxy.

8. Procédé selon la revendication 7, qui est **caractérisé en ce que** : le composé de Formule VIII est obtenu en éliminant le groupe protecteur acétyle du composé de Formule VII en présence d'un réactif de désacétylation : 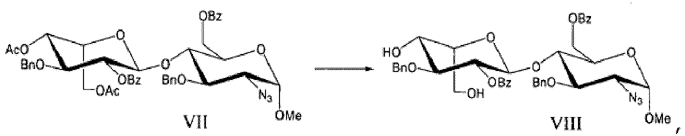 de préférence, le réactif de désacétylation est choisi parmi une solution de chlorure d'hydrogène en méthanol, une solution de chlorure d'hydrogène dans de l'éthanol, une solution de triéthylamine dans du méthanol, une solution de triéthylamine dans de l'éthanol, le méthoxyde de sodium ou l'éthoxyde de sodium.

9. Procédé selon la revendication 8, qui est **caractérisé en ce que** : le composé de Formule VII est obtenu par la réaction de glycosylation du composé de Formule XI et du composé de Formule XII dans la condition qui permet la formation de liaison glucosique :

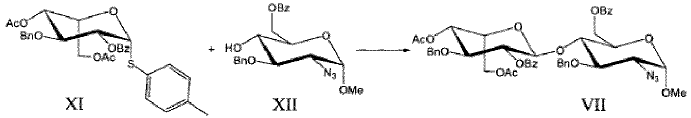

10. Procédé selon la revendication 9, qui est **caractérisé en ce que** : le composé de Formule XI est obtenu en faisant réagir le composé de formule XIII avec un réactif d'acétylation en condition alcaline afin de protéger les 2 groupes hydroxy exposés avec le groupe acétyle : 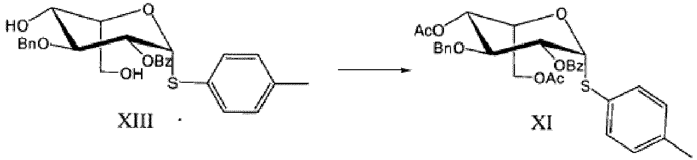 de préférence, la base est choisie parmi une base organique ou une base inorganique, dans lequel la base organique est choisie parmi la pyridine ou la triéthylamine, et la base inorganique est choisie parmi le carbonate de potassium, le carbonate de sodium ou le bicarbonate de sodium ; le réactif d'acétylation est choisi parmi l'anhydride acétique ou le chlorure d'acétyle.

11. Procédé selon la revendication 9, qui est **caractérisé en ce que** : le procédé de préparation pour le composé de Formule XII comprend les étapes suivantes :
1) le composé de Formule XIV est traité par une base forte afin de donner le composé de Formule XV en éliminant le groupe carbobenzoxy (-Cbz), et en exposant le groupe amino : 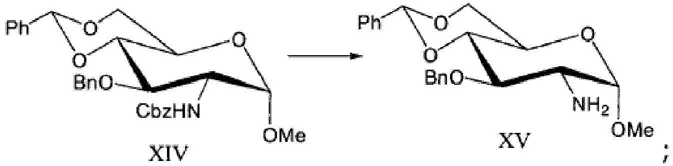
2) le composé de Formule XV est mis à réagir avec un réactif de transfert diazo afin de donner le composé de Formule XVI en convertissant les groupes amino exposés dans le composé de Formule XV en le groupe azido : 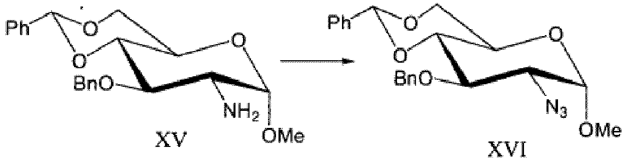
3) le benzylidène est éliminé du composé de Formule XVI en condition acide pour donner le composé de Formule XVII : 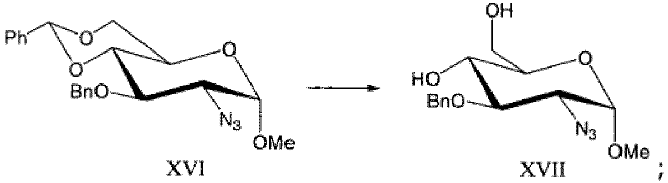
4) le composé de Formule XVII est mis à réagir avec un réactif de benzoylation en condition alcaline pour donner le composé de Formule XII en protégeant le groupe hydroxyle alcoolique primaire avec le groupe benzoyle :
